# EUROPEAN PATENT APPLICATION

(11) **EP 3 431 993 A1**
(43) Date of publication of application: **23.01.2019**
(21) Application number: 18174402.0
(22) Date of filing: 19.12.2011
(51) Int. Cl.: G01N 33/53, A61K 39/395, A61K 38/17, C07K 16/28, G01N 33/68, A61K 39/00

(54) **MODULATING AGONISTIC TNFR ANTIBODIES**

(30) Priority: 20.12.2010 US 201061424996 P
(62) Divisional of application: 11850401.8
(71) Applicant: The Rockefeller University, New York, NY 10021 (US)
(72) Inventor: RAVETCH, Jeffrey, V., New York, NY 10022 (US); LI, Fubin, New York, NY 10065 (US)
(74) Representative: Barker Brettell LLP

(57) **Abstract**

The instant invention relates to agents (e.g., agonistic antibodies) able to stimulate the immune system of a mammalian animal and activate target-cell specific T lymphocyte responses. Such agents may be identified based on the ability to engage a receptor from the TNFR Superfamily and thereby mimic the natural ligand for the receptor from the TNFR Superfamily. Modified antibodies of this class display enhanced immunostimulatory activity and may be formulated and administered for the treatment of a disease or disorder.

SEQUENCE LISTING

<110>  THE ROCKEFELLER UNIVERSITY

<120>  MODULATING AGONISTIC TNFR ANTIBODIES

<130>  JA68431P.EPPD1

<150>  EP11850401.8
<151>  2011-12-19

<150>  PCT/US11/65830
<151>  2011-12-19

<150>  61/424,996
<151>  2010-12-20

<160>  84

<170>  PatentIn version 3.5

<210>  1
<211>  465
<212>  PRT
<213>  Homo sapiens

<400>  1

Met Asp Ile Arg Leu Ser Leu Val Phe Leu Val Leu Phe Ile Lys Gly
1               5                   10                  15

Val Gln Cys Glu Val Gln Leu Val Glu Ser Asp Gly Gly Leu Val Gln
            20                  25                  30

Pro Gly Arg Ser Leu Lys Leu Pro Cys Ala Ala Ser Gly Phe Thr Phe
        35                  40                  45

Ser Asp Tyr Tyr Met Ala Trp Val Arg Gln Ala Pro Thr Lys Gly Leu
    50                  55                  60

Glu Trp Val Ala Ser Ile Ser Tyr Asp Gly Ser Ser Thr Tyr Tyr Arg
65                  70                  75                  80

Asp Ser Val Lys Gly Arg Phe Thr Ile Ser Arg Asp Asn Ala Lys Ser
                85                  90                  95

Thr Leu Tyr Leu Gln Met Asp Ser Leu Arg Ser Glu Asp Thr Ala Thr
            100                 105                 110

Tyr Tyr Cys Gly Arg His Ser Ser Tyr Phe Asp Tyr Trp Gly Gln Gly
        115                 120                 125

Val Met Val Thr Val Ser Ser Ala Ser Thr Lys Gly Pro Ser Val Phe
    130                 135                 140

Pro Leu Ala Pro Ser Ser Lys Ser Thr Ser Gly Gly Thr Ala Ala Leu
145                 150                 155                 160

Gly Cys Leu Val Lys Asp Tyr Phe Pro Glu Pro Val Thr Val Ser Trp
                165                 170                 175

Asn Ser Gly Ala Leu Thr Ser Gly Val His Thr Phe Pro Ala Val Leu
            180                 185                 190

Gln Ser Ser Gly Leu Tyr Ser Leu Ser Ser Val Val Thr Val Pro Ser
        195                 200                 205

Ser Ser Leu Gly Thr Gln Thr Tyr Ile Cys Asn Val Asn His Lys Pro
    210                 215                 220

Ser Asn Thr Lys Val Asp Lys Arg Val Glu Pro Lys Ser Cys Asp Lys
225                 230                 235                 240

Thr His Thr Cys Pro Pro Cys Pro Ala Pro Glu Leu Leu Gly Gly Pro
                245                 250                 255

Ser Val Phe Leu Phe Pro Pro Lys Pro Lys Asp Thr Leu Met Ile Ser
            260                 265                 270

Arg Thr Pro Glu Val Thr Cys Val Val Val Asp Val Ser His Glu Asp
        275                 280                 285

Pro Glu Val Lys Phe Asn Trp Tyr Val Asp Gly Val Glu Val His Asn
    290                 295                 300

Ala Lys Thr Lys Pro Arg Glu Glu Gln Tyr Asn Ser Thr Tyr Arg Val
305                 310                 315                 320

Val Ser Val Leu Thr Val Leu His Gln Asp Trp Leu Asn Gly Lys Glu
                325                 330                 335

Tyr Lys Cys Lys Val Ser Asn Lys Ala Leu Pro Ala Pro Ile Glu Lys
            340                 345                 350

Thr Ile Ser Lys Ala Lys Gly Gln Pro Arg Glu Pro Gln Val Tyr Thr
        355                 360                 365

Leu Pro Pro Ser Arg Asp Glu Leu Thr Lys Asn Gln Val Ser Leu Thr
    370                 375                 380

Cys Leu Val Lys Gly Phe Tyr Pro Ser Asp Ile Ala Val Glu Trp Glu
385                 390                 395                 400

Ser Asn Gly Gln Pro Glu Asn Asn Tyr Lys Thr Thr Pro Pro Val Leu
                405                 410                 415

Asp Ser Asp Gly Ser Phe Phe Leu Tyr Ser Lys Leu Thr Val Asp Lys
            420                 425                 430

Ser Arg Trp Gln Gln Gly Asn Val Phe Ser Cys Ser Val Met His Glu
        435                 440                 445

Ala Leu His Asn His Tyr Thr Gln Lys Ser Leu Ser Leu Ser Pro Gly
    450                 455                 460

Lys
465

<210> 2
<211> 233
<212> PRT
<213> Homo sapiens

<400> 2

Met Glu Thr Asp Arg Leu Leu Leu Trp Val Leu Leu Leu Trp Val Pro
1               5                   10                  15

Gly Ser Thr Gly Asp Thr Val Leu Thr Gln Ser Pro Ala Leu Ala Val
            20                  25                  30

Ser Pro Gly Glu Arg Val Thr Ile Ser Cys Arg Ala Ser Asp Ser Val
        35                  40                  45

Ser Thr Leu Met His Trp Tyr Gln Gln Lys Pro Gly Gln Gln Pro Lys
    50                  55                  60

Leu Leu Ile Tyr Leu Ala Ser His Leu Glu Ser Gly Val Pro Ala Arg
65                  70                  75                  80

Phe Ser Gly Ser Gly Ser Gly Thr Asp Phe Thr Leu Thr Ile Asp Pro
                85                  90                  95

Val Glu Ala Asp Asp Thr Ala Thr Tyr Tyr Cys Gln Gln Ser Trp Asn
            100                 105                 110

Asp Pro Trp Thr Phe Gly Gly Gly Thr Lys Leu Glu Leu Lys Arg Thr
        115                 120                 125

Val Ala Ala Pro Ser Val Phe Ile Phe Pro Pro Ser Asp Glu Gln Leu
    130                 135                 140

Lys Ser Gly Thr Ala Ser Val Val Cys Leu Leu Asn Asn Phe Tyr Pro
145                 150                 155                 160

Arg Glu Ala Lys Val Gln Trp Lys Val Asp Asn Ala Leu Gln Ser Gly
                165                 170                 175

Asn Ser Gln Glu Ser Val Thr Glu Gln Asp Ser Lys Asp Ser Thr Tyr
            180                 185                 190

Ser Leu Ser Ser Thr Leu Thr Leu Ser Lys Ala Asp Tyr Glu Lys His
        195                 200                 205

Lys Val Tyr Ala Cys Glu Val Thr His Gln Gly Leu Ser Ser Pro Val
    210                 215                 220

Thr Lys Ser Phe Asn Arg Gly Glu Cys
225                 230

<210>  3
<211>  238
<212>  PRT
<213>  Homo sapiens

<400>  3

Lys Val Asp Lys Arg Val Glu Pro Lys Ser Cys Asp Lys Thr His Thr
1               5                   10                  15

Cys Pro Pro Cys Pro Ala Pro Glu Leu Leu Gly Gly Pro Ser Val Phe
            20                  25                  30

Leu Phe Pro Pro Lys Pro Lys Asp Thr Leu Met Ile Ser Arg Thr Pro
        35                  40                  45

Glu Val Thr Cys Val Val Val Asp Val Ser His Glu Asp Pro Glu Val
    50                  55                  60

Lys Phe Asn Trp Tyr Val Asp Gly Val Glu Val His Asn Ala Lys Thr
65                  70                  75                  80

Lys Pro Arg Glu Glu Gln Tyr Asn Ser Thr Tyr Arg Val Val Ser Val
                85                  90                  95

Leu Thr Val Leu His Gln Asp Trp Leu Asn Gly Lys Glu Tyr Lys Cys
            100                 105                 110

Lys Val Ser Asn Lys Ala Leu Pro Ala Pro Ile Glu Lys Thr Ile Ser
        115                 120                 125

Lys Ala Lys Gly Gln Pro Arg Glu Pro Gln Val Tyr Thr Leu Pro Pro
    130                 135                 140

Ser Arg Asp Glu Leu Thr Lys Asn Gln Val Ser Leu Thr Cys Leu Val
145                 150                 155                 160

Lys Gly Phe Tyr Pro Ser Asp Ile Ala Val Glu Trp Glu Ser Asn Gly
                165                 170                 175

Gln Pro Glu Asn Asn Tyr Lys Thr Thr Pro Pro Val Leu Asp Ser Asp
            180                 185                 190

Gly Ser Phe Phe Leu Tyr Ser Lys Leu Thr Val Asp Lys Ser Arg Trp
        195                 200                 205

Gln Gln Gly Asn Val Phe Ser Cys Ser Val Met His Glu Ala Leu His
    210                 215                 220

Asn His Tyr Thr Gln Lys Ser Leu Ser Leu Ser Pro Gly Lys
225                 230                 235

<210>  4
<211>  238
<212>  PRT
<213>  artificial

<220>
<223>  Modified Fc region of IgG1 with S267E

<400>  4

Lys Val Asp Lys Arg Val Glu Pro Lys Ser Cys Asp Lys Thr His Thr
1               5                   10                  15

Cys Pro Pro Cys Pro Ala Pro Glu Leu Leu Gly Gly Pro Ser Val Phe
            20                  25                  30

Leu Phe Pro Pro Lys Pro Lys Asp Thr Leu Met Ile Ser Arg Thr Pro
        35                  40                  45

Glu Val Thr Cys Val Val Val Asp Val Glu His Glu Asp Pro Glu Val
    50                  55                  60

Lys Phe Asn Trp Tyr Val Asp Gly Val Glu Val His Asn Ala Lys Thr
65                  70                  75                  80

Lys Pro Arg Glu Glu Gln Tyr Asn Ser Thr Tyr Arg Val Val Ser Val
                85                  90                  95

Leu Thr Val Leu His Gln Asp Trp Leu Asn Gly Lys Glu Tyr Lys Cys
            100                 105                 110

Lys Val Ser Asn Lys Ala Leu Pro Ala Pro Ile Glu Lys Thr Ile Ser
        115                 120                 125

Lys Ala Lys Gly Gln Pro Arg Glu Pro Gln Val Tyr Thr Leu Pro Pro
    130                 135                 140

Ser Arg Asp Glu Leu Thr Lys Asn Gln Val Ser Leu Thr Cys Leu Val
145                 150                 155                 160

Lys Gly Phe Tyr Pro Ser Asp Ile Ala Val Glu Trp Glu Ser Asn Gly
                165                 170                 175

Gln Pro Glu Asn Asn Tyr Lys Thr Thr Pro Pro Val Leu Asp Ser Asp
            180                 185                 190

Gly Ser Phe Phe Leu Tyr Ser Lys Leu Thr Val Asp Lys Ser Arg Trp
        195                 200                 205

Gln Gln Gly Asn Val Phe Ser Cys Ser Val Met His Glu Ala Leu His
    210                 215                 220

Asn His Tyr Thr Gln Lys Ser Leu Ser Leu Ser Pro Gly Lys
225                 230                 235

<210>  5
<211>  5
<212>  PRT
<213>  Homo sapiens

<400>  5

Asp Tyr Tyr Met Ala
1               5

<210>  6
<211>  17
<212>  PRT
<213>  Homo sapiens

<400>  6

Ser Ile Ser Tyr Asp Gly Ser Ser Thr Tyr Tyr Arg Asp Ser Val Lys
1               5                   10                  15

Gly

<210>  7
<211>  7
<212>  PRT
<213>  Homo sapiens

<400>  7

His Ser Ser Tyr Phe Asp Tyr
1               5


<210>  8
<211>  11
<212>  PRT
<213>  Homo sapiens

<400>  8

Arg Ala Ser Asp Ser Val Ser Thr Leu Met His
1               5                   10


<210>  9
<211>  7
<212>  PRT
<213>  Homo sapiens

<400>  9

Leu Ala Ser His Leu Glu Ser
1               5


<210>  10
<211>  9
<212>  PRT
<213>  Homo sapiens

<400>  10

Gln Gln Ser Trp Asn Asp Pro Trp Thr
1               5


<210>  11
<211>  41
<212>  DNA
<213>  Artificial

<220>
<223>  Synthetic Primer

<400>  11
cctcggttct atcgattgaa ttccaccatg ggatggtcat g                          41


<210>  12
<211>  35
<212>  DNA
<213>  Artificial

<220>
<223>  Synthetic Primer

<400>  12
ttggtggagg ctgaggagac tgtgaccatg actcc                                35

<210>  13
<211>  36
<212>  DNA
<213>  Artificial

<220>
<223>  Synthetic Primer

<400>  13
ggtcacagtc tcctcagcct ccaccaaggg cccatc                               36

<210>  14
<211>  41
<212>  DNA
<213>  Artificial

<220>
<223>  Synthetic Primer

<400>  14
cttggccatg tcgctagctt tacccggaga cagggagagg c                         41

<210>  15
<211>  41
<212>  DNA
<213>  Artificial

<220>
<223>  Synthetic Primer

<400>  15
cctcggttct atcgattgaa ttccaccatg ggatggtcat g                         41

<210>  16
<211>  37
<212>  DNA
<213>  Artificial

<220>
<223>  Synthetic Primer

<400>  16
cagccacagt tcgtttcaat tccagcttgg tgcctcc                              37

<210>  17
<211>  35
<212>  DNA
<213>  Artificial

<220>
<223>  Synthetic Primer

<400>  17
gctggaattg aaacgaactg tggctgcacc atctg                                35

<210>  18
<211>  48
<212>  DNA
<213>  Artificial

<220>
<223>  Synthetic Primer

<400>  18
caagcttggg agcggccgcc taacactctc ccctgttgaa gctctttg                    48


<210>  19
<211>  41
<212>  DNA
<213>  Artificial

<220>
<223>  Synthetic Primer

<400>  19
cctcggttct atcgattgaa ttccaccatg ggatggtcat g                           41


<210>  20
<211>  36
<212>  DNA
<213>  Artificial

<220>
<223>  Synthetic Primer

<400>  20
cttggtggag gctgaggaga ctgtgaccat gactcc                                 36


<210>  21
<211>  36
<212>  DNA
<213>  Artificial

<220>
<223>  Synthetic Primer

<400>  21
ggtcacagtc tcctcagcct ccaccaaggg cccatc                                 36


<210>  22
<211>  41
<212>  DNA
<213>  Artificial

<220>
<223>  Synthetic Primer

<400>  22
cttggccatg tcgctagctt tacccggaga cagggagagg c                           41


<210>  23

<211>  41
<212>  DNA
<213>  Artificial

<220>
<223>  Synthetic Primer

<400>  23
cctcggttct atcgattgaa ttccaccatg ggatggtcat g                41


<210>  24
<211>  37
<212>  DNA
<213>  Artificial

<220>
<223>  Synthetic Primer

<400>  24
cagccacagt tcgtttcagt tccagcttgg tcccagg                37


<210>  25
<211>  35
<212>  DNA
<213>  Artificial

<220>
<223>  Synthetic Primer

<400>  25
gctggaactg aaacgaactg tggctgcacc atctg                35


<210>  26
<211>  48
<212>  DNA
<213>  Artificial

<220>
<223>  Synthetic Primer

<400>  26
caagcttggg agcggccgcc taacactctc ccctgttgaa gctctttg                48


<210>  27
<211>  21
<212>  DNA
<213>  Artificial

<220>
<223>  Synthetic Primer

<400>  27
acaaggattg cattcccttg g                21


<210>  28
<211>  21
<212>  DNA
<213>  Artificial

<220>
<223>  Synthetic Primer

<400>  28
cttgtccacc ttggtgctgc t                                                21


<210>  29
<211>  27
<212>  DNA
<213>  Artificial

<220>
<223>  Synthetic Primer

<400>  29
ctcattcctg ttgaagctct tgacgac                                          27


<210>  30
<211>  23
<212>  DNA
<213>  Artificial

<220>
<223>  Synthetic Primer

<400>  30
gggtgaggat gatgtcttat gaa                                              23


<210>  31
<211>  39
<212>  DNA
<213>  Artificial

<220>
<223>  Synthetic Primer

<400>  31
cgattgaatt ccaccatgga catcaggctc agcttggtt                             39


<210>  32
<211>  38
<212>  DNA
<213>  Artificial

<220>
<223>  Synthetic Primer

<400>  32
gcccttggtg gtggctgagg agactgtgac catgactc                              38


<210>  33
<211>  39
<212>  DNA
<213>  Artificial

<220>
<223>  Synthetic Primer

<400>  33
gtcacagtct cctcagccac caccaagggc ccatctgtc                              39


<210>  34
<211>  43
<212>  DNA
<213>  Artificial

<220>
<223>  Synthetic Primer

<400>  34
cttgggagcg gccgctcatt taccaggaga gtgggagagg ctc                         43


<210>  35
<211>  39
<212>  DNA
<213>  Artificial

<220>
<223>  Synthetic Primer

<400>  35
cgattgaatt ccaccatgga catcaggctc agcttggtt                              39


<210>  36
<211>  38
<212>  DNA
<213>  Artificial

<220>
<223>  Synthetic Primer

<400>  36
ggctgttgtt ttggctgagg agactgtgac catgactc                               38


<210>  37
<211>  39
<212>  DNA
<213>  Artificial

<220>
<223>  Synthetic Primer

<400>  37
gtcacagtct cctcagccaa aacaacagcc ccatcggtc                              39


<210>  38
<211>  43
<212>  DNA
<213>  Artificial

<220>
<223>  Synthetic Primer

<400>  38
cttgggagcg gccgctcatt tacccagaga ccgggagatg gtc                         43

<210>  39
<211>  39
<212>  DNA
<213>  Artificial

<220>
<223>  Synthetic Primer

<400>  39
cgattgaatt ccaccatgga catcaggctc agcttggtt                              39


<210>  40
<211>  38
<212>  DNA
<213>  Artificial

<220>
<223>  Synthetic Primer

<400>  40
gcccttggtg gaggctgagg agactgtgac catgactc                              38


<210>  41
<211>  39
<212>  DNA
<213>  Artificial

<220>
<223>  Synthetic Primer

<400>  41
gtcacagtct cctcagcctc caccaagggc ccatcggtc                             39


<210>  42
<211>  43
<212>  DNA
<213>  Artificial

<220>
<223>  Synthetic Primer

<400>  42
cttgggagcg gccgctcatt tacccggaga cagggagagg ctc                        43


<210>  43
<211>  39
<212>  DNA
<213>  Artificial

<220>
<223>  Synthetic Primer

<400>  43
cgattgaatt ccaccatgga gacagacaga ctcctgcta                             39


<210>  44

<211>  38
<212>  DNA
<213>  Artificial

<220>
<223>  Synthetic Primer

<400>  44
gcagcatcag cccgtgagga gactgtgacc atgactcc                              38


<210>  45
<211>  40
<212>  DNA
<213>  Artificial

<220>
<223>  Synthetic Primer

<400>  45
caagctggaa ttgaaacggg ctgatgctgc accaactgta                            40


<210>  46
<211>  43
<212>  DNA
<213>  Artificial

<220>
<223>  Synthetic Primer

<400>  46
cttgggagcg gccgctcaac actcattcct gttgaagctc ttg                        43


<210>  47
<211>  39
<212>  DNA
<213>  Artificial

<220>
<223>  Synthetic Primer

<400>  47
cgattgaatt ccaccatgga gacagacaga ctcctgcta                             39


<210>  48
<211>  38
<212>  DNA
<213>  Artificial

<220>
<223>  Synthetic Primer

<400>  48
gcagccacag ttcgtgagga gactgtgacc atgactcc                              38


<210>  49
<211>  40
<212>  DNA
<213>  Artificial

<220>
<223>  Synthetic Primer

<400>  49
caagctggaa ttgaaacgaa ctgtggctgc accatctgtc                              40


<210>  50
<211>  44
<212>  DNA
<213>  Artificial

<220>
<223>  Synthetic Primer

<400>  50
cttgggagcg gccgctcaac actctcccct gttgaagctc tttg                         44


<210>  51
<211>  27
<212>  DNA
<213>  Artificial

<220>
<223>  Synthetic Primer

<400>  51
gtggtggacg tggaacacga agaccct                                            27


<210>  52
<211>  27
<212>  DNA
<213>  Artificial

<220>
<223>  Synthetic Primer

<400>  52
agggtcttcg tgttccacgt ccaccac                                            27


<210>  53
<211>  33
<212>  DNA
<213>  Artificial

<220>
<223>  Synthetic Primer

<400>  53
cctgaactcc tggacggacc gtcagtcttc ctc                                     33


<210>  54
<211>  33
<212>  DNA
<213>  Artificial

<220>
<223>  Synthetic Primer

<400>  54
gaggaagact gacggtccgt ccaggagttc agg                                    33


<210>  55
<211>  27
<212>  DNA
<213>  Artificial

<220>
<223>  Synthetic Primer

<400>  55
   ctggggggac cggatgtctt cctcttc                                            27


<210>  56
<211>  27
<212>  DNA
<213>  Artificial

<220>
<223>  Synthetic Primer

<400>  56
gaagaggaag acatccggtc cccccag                                           27


<210>  57
<211>  27
<212>  DNA
<213>  Artificial

<220>
<223>  Synthetic Primer

<400>  57
gtctccaaca aagccttccc agccccc                                           27


<210>  58
<211>  27
<212>  DNA
<213>  Artificial

<220>
<223>  Synthetic Primer

<400>  58
gggggctggg aaggctttgt tggagac                                           27


<210>  59
<211>  27
<212>  DNA
<213>  Artificial

<220>
<223>  Synthetic Primer

<400>  59
   ctggggggac cggatgtctt cctcttc                                            27

<210>  60
<211>  27
<212>  DNA
<213>  Artificial

<220>
<223>  Synthetic Primer

<400>  60
gaagaggaag acatccggtc cccccag                                          27


<210>  61
<211>  33
<212>  DNA
<213>  Artificial

<220>
<223>  Synthetic Primer

<400>  61
gccctcccag cccccgaaga gaaaaccatc tcc                                   33


<210>  62
<211>  33
<212>  DNA
<213>  Synthetic Primer

<400>  62
ggagatggtt ttctcttcgg gggctgggag ggc                                   33


<210>  63
<211>  38
<212>  DNA
<213>  Artificial

<220>
<223>  Synthetic Primer

<400>  63
aattgtcgac caccatggtg tctttgcctc ggctgtgc                              38


<210>  64
<211>  37
<212>  DNA
<213>  Artificial

<220>
<223>  Synthetic Primer

<400>  64
aattgcggcc gctcagacca ggggcctcaa ggctatg                               37


<210>  65
<211>  24
<212>  DNA
<213>  Artificial

<220>
<223>  Synthetic Primer

<400>  65
gctcacgtcc accaccacac atgt                                              24


<210>  66
<211>  24
<212>  DNA
<213>  Artificial

<220>
<223>  Synthetic Primer

<400>  66
gaaatagccc ttgaccaggc atcc                                              24


<210>  67
<211>  28
<212>  DNA
<213>  Artificial

<220>
<223>  Synthetic Primer

<400>  67
ctaacactca ttcctgttca gggtcttg                                          28


<210>  68
<211>  24
<212>  DNA
<213>  Artificial

<220>
<223>  Synthetic Primer

<400>  68
gctgctcagg ctgtaggtgc tgtc                                              24


<210>  69
<211>  42
<212>  DNA
<213>  Artificial

<220>
<223>  Synthetic Primer

<400>  69
cgattgaatt ccaccatgag actgctgggt cttctgtacc tg                          42


<210>  70
<211>  39
<212>  DNA
<213>  Artificial

<220>
<223>  Synthetic Primer

<400>  70
gcccttggtg gaggctgagg agacggtgac cagggtccc                              39


<210>  71
<211>  39
<212>  DNA
<213>  Artificial

<220>
<223>  Synthetic Primer

<400>  71
gtcaccgtct cctcagcctc caccaagggc ccatcggtc                              39


<210>  72
<211>  43
<212>  DNA
<213>  Artificial

<220>
<223>  Synthetic Primer

<400>  72
cttgggagcg gccgctcatt tacccggaga cagggagagg ctc                         43


<210>  73
<211>  38
<212>  DNA
<213>  Artificial

<220>
<223>  Synthetic Primer

<400>  73
cgattgaatt ccaccatggc catgaaggtt cctgctca                               38


<210>  74
<211>  38
<212>  DNA
<213>  Artificial

<220>
<223>  Synthetic Primer

<400>  74
gcagccacag ttcgtttgat ttccagtctg gtccctcc                               38


<210>  75
<211>  40
<212>  DNA
<213>  Artificial

<220>
<223>  Synthetic Primer

<400>  75
cagactggaa atcaaacgaa ctgtggctgc accatctgtc                             40

<210>  76
<211>  43
<212>  DNA
<213>  Artificial

<220>
<223>  Synthetic Primer

<400>  76
ttgggagcgg ccgctcaaca ctctcccctg ttgaagctct ttg                    43


<210>  77
<211>  470
<212>  PRT
<213>  Homo sapiens

<400>  77

<210>  78
<211>  236
<212>  PRT
<213>  Homo sapiens

<400>  78

Met Ala Met Lys Val Pro Ala Gln Ala Leu Val Ile Leu Leu Leu Trp
1               5                   10                  15

Val Ser Gly Ala Thr Cys Asp Ile Gln Val Thr Gln Ser Pro Ser Leu
            20                  25                  30

Leu Ser Ala Ser Phe Gly Asp Lys Val Thr Ile Asn Cys Leu Val Thr
        35                  40                  45

Gln Asp Ile Thr Tyr Tyr Leu Ser Trp Tyr Gln Gln Lys Ser Gly Gln
    50                  55                  60

Pro Pro Thr Leu Leu Ile Tyr Asn Gly Asn Ser Leu Gln Ser Gly Val
65                  70                  75                  80

Pro Ser Arg Phe Ser Gly Gln Tyr Ser Gly Arg Thr Phe Thr Leu Ser
                85                  90                  95

Leu Ser Ser Leu Glu Pro Glu Asp Ala Gly Thr Tyr Tyr Cys Leu Gln
            100                 105                 110

His Tyr Ser Val Pro Phe Thr Phe Gly Gly Gly Thr Arg Leu Glu Ile
        115                 120                 125

Lys Arg Thr Val Ala Ala Pro Ser Val Phe Ile Phe Pro Pro Ser Asp
    130                 135                 140

Glu Gln Leu Lys Ser Gly Thr Ala Ser Val Val Cys Leu Leu Asn Asn
145                 150                 155                 160

Phe Tyr Pro Arg Glu Ala Lys Val Gln Trp Lys Val Asp Asn Ala Leu

165                 170                 175

Gln Ser Gly Asn Ser Gln Glu Ser Val Thr Glu Gln Asp Ser Lys Asp
            180                 185                 190

Ser Thr Tyr Ser Leu Ser Ser Thr Leu Thr Leu Ser Lys Ala Asp Tyr
        195                 200                 205

Glu Lys His Lys Val Tyr Ala Cys Glu Val Thr His Gln Gly Leu Ser
    210                 215                 220

Ser Pro Val Thr Lys Ser Phe Asn Arg Gly Glu Cys
225                 230                 235

<210>  79
<211>  7
<212>  PRT
<213>  Homo sapiens

<400>  79

Ala Gly Gly Tyr Trp Trp Thr
1               5

<210>  80
<211>  16
<212>  PRT
<213>  Homo sapiens

<400>  80

Tyr Ile Tyr Ser Ser Gly Ser Thr Asn Tyr Asn Pro Ser Ile Lys Ser
1               5                   10                  15

<210>  81
<211>  11
<212>  PRT
<213>  Homo sapiens

<400>  81

Ala Gly Thr Ser Tyr Ser Gly Phe Phe Asp Ser
1               5                   10

<210>  82
<211>  11
<212>  PRT
<213>  Homo sapiens

<400>  82

Leu Val Thr Gln Asp Ile Thr Tyr Tyr Leu Ser
1               5                   10

<210> 83
<211> 7
<212> PRT
<213> Homo sapiens

<400> 83

Asn Gly Asn Ser Leu Gln Ser
1               5


<210> 84
<211> 9
<212> PRT
<213> Homo sapiens

<400> 84

Leu Gln His Tyr Ser Val Pro Phe Thr
1               5

## Description

### CROSS REFERENCE TO RELATED APPLICATION

This application claims priority of U.S. Provisional Application No. 61/424,996, filed on December 20, 2010. The content of the application is incorporated herein by reference in its entirety.

### STATEMENT REGARDING FEDERALLY FUNDED RESEARCH

The invention disclosed herein was made, at least in part, with Government support under National Institutes of Health Grant No. CA 080757. Accordingly, the U.S. Government has certain rights in this invention.

### FIELD OF THE INVENTION

The present invention relates to agents (such as modified antibodies) and associated methods and compositions that both engage a receptor from the TNFR Superfamily and enhance its agonistic activities (including but not limited to, adjuvant activities, immunestimulatory and apoptosis-inducing activities, and abilities to activate tumor specific T cell responses).

### BACKGROUND OF THE INVENTION

Certain antibody functions are mediated by Fc receptors (FcRs), which bind the Fc region of the antibody and are defined by their specificity for immunoglobulin isotypes. One important family of cell surface Fc receptors for the IgG class of antibodies are Fc gamma receptors (FcγRs), which are understood to mediate communication between antibodies and the cellular arm of the immune system. FcγRs are expressed in a variety of immune cells including monocytes, macrophages, neutrophils, dendritic cells, eosinophils, mast cells, platelets, B cells, large granular lymphocytes, Langerhans' cells, and natural killer (NK) cells. Formation of the Fc/FcγR complex is thought to recruit effector cells to sites of bound antigen, typically resulting in signaling events within the cells and important subsequent immune responses such as release of inflammation mediators, T cell activation, B cell activation, endocytosis, phagocytosis, and/or cytotoxic attack. For example, FcγRs are known to mediate cytotoxic and phagocytic effector functions, which are understood to be mechanisms by which antibodies destroy targeted cells. Antibody dependent cell-mediated cytoxicity (ADCC) is one such function where nonspecific cytotoxic cells expressing FcγRs recognize bound antibody on a target cell and subsequently cause lysis of the target cell. Antibody dependent cell-mediated phagocytosis (ADCP) provides similar cell-mediated reactivity where nonspecific cytotoxic cells recognize bound antibody and subsequently cause phagocytosis of the target cell.

In humans, the FcγR protein family includes FcγRI (including isoforms FcγRIa, FcγRIb, and FcγRIc); FcγRII (including isoforms FcγRIIa, FcγRIIb, and FcγRIIc); and FcγRIII (including isoforms FcγRIIIa and FcγRIIIb). Subclasses of IgG antibodies are known to have different affinities for these FcγRs and, as a result, elicit different responses. In the context of cytotoxic and phagocytic effector cell functionality, FcγRI, FcγRIIa/c, and FcγRIIIa are thought of as positive regulators of immune complex-triggered activation. FcγRIIb, however, is typically thought of as inhibitory, i.e. an inhibitor of cytotoxicity and/or phagocytotoxicity. Such features provide value for the use of such antibodies in a therapeutic context, in particular, for treatment methods where immunological responsiveness or targeted cell death is critical. Monoclonal antibodies or other therapeutic antibodies, therefore, can be engineered with a Fab region targeting an epitope on the cell of interest and a Fc region adapted to improve the immunological responsiveness, i.e. ADCC, ADCP, complement, cell apoptosis, etc (See, e.g., U.S. Patent No. 7,317,791).

While there are numerous possibilities for such a therapeutic design, improvement of anti-tumor efficacy presents one of the more attractive being explored. In particular, by targeting one or more known cell "death receptors," researchers have studied the use of the foregoing FcγR-dependent mechanism to modulate immunological response mechanisms that would trigger tumor cell death. With B-lineage neoplasms, for example, one avenue of approach as been to target a tumor necrosis factor receptor (TNFR). Many antibodies developed to date that bind to such receptors also bind preferentially to FcγRIII and/or FcγRIa, and minimally to FcγRIIb. In Horton, et al. Blood, Jul 2010; doi:10.1182/blood-2010-01-265280, for example, a humanized anti-CD40 antibody was disclosed that was Fc-engineered for increased binding to FcγRIIIa and FcγRIa. Consistent with the foregoing, this was shown to maximize the secondary cell ADCC- or ADCP-dependent responsiveness. While promising as a strategy, the use of such independent pathways has a relatively non-specific component, the mechanism of which is not well understood. Thus, ADCC- and ADCP-based strategies may exhibit efficacy, safety and toxicity concerns, which would make them unfit for therapeutic administration.

Another approach is to elicit cell apoptosis using non-ADCC or non-ADPC pathways. In Xu, et al., J. Immunol., 2003, 171(2):562-8, for example, researchers used the murine-Fas targeting antibodies Jo2 and HFE7A to demonstrate that FcγRs modulate the agonistic activities of anti-Fas antibodies. With respect to Jo2, it was determined that FcγRIIb ligand binding, while providing inhibitory activity of ADCC-based mechanism, also resulted in increased apoptosis of the target cell. Xu, *et al.* leaves unanswered, however, the exact immunological mechanism that facilitated cell death and what role, direct or otherwise, the FcγRIIb ligand played in that mechanism. Moreover, other members of the TNFR superfamily, such as CD40, a cell surface receptor with pleitropic activities including potent immune stimulatory capacity, were not investigated.

Accordingly, there remains a continuing need for the development of antibodies with enhanced therapeutic properties, while reducing the risk for toxicity to the patient. In particular, there remains a continuing need for the development of antibodies that are engineered to bivalently target cell specific antigens and mimic or enhance native ligands to act as direct adjuvants, immuno-stimulatory agents, or agents with other agonistic activities for therapeutic responsiveness. Such antibodies can be use for a host of therapeutic functions including, but not limited to, anti-cancer therapeutics.

### SUMMARY OF THE INVENTION

The present invention fills at least the foregoing needs by providing an advantageous strategy for stimulating or enhancing natural immunological responsiveness by providing for agents, such as antibody Fc variants, with preferential and/or selective binding to the inhibitory FcγR receptor and/or TNFR superfamily receptor.

In one aspect, the invention features a method of identifying an agent useful as an adjuvant or an apoptotic agent. The method includes obtaining a test agent; evaluating a first ability of the test agent to bind to a TNFR superfamily receptor; and evaluating a second ability of the test agent to bind to an inhibitory Fcγ receptor (FcγR), wherein the abilities of the test agent to bind both the TNFR superfamily receptor and the inhibitory FcγR indicate that the test agent is a candidate for an adjuvant or an apoptotic agent. The TNFR superfamily receptor can contain (i) one or more TNF receptor-associated factors (TRAF) interacting motifs or (ii) one or more death domains. These two types of TNFRs can be used to identify candidates for adjuvants and candidates for apoptotic agents, respectively. Examples of these two types of TNFR are CD40 and DR5, respectively. The inhibitory Fcγ receptor can be human or mouse FcγRIIb. The test agent can contain a polypeptide, such as one having an Fc region of an antibody. In that case, the method may also include testing an Fc region of the antibody for binding affinity and/or selective binding affinity to FcγRIIb. Such binding affinities and/or selectivity acts as a predictor of enhanced *in vivo* T-lymphocyte responsiveness and reduced ADCC. The foregoing test methods while not limited thereto, may be carried out in an FcγRIIb humanized mouse and comprises measurement of T cell stimulation as compared to a native or parent antibody. In one example, the test agent can be an antibody (e.g., anti-CD40 antibodies) or a fusion polypeptide.

The above method can further include examining a third ability of the test agent to bind to an activatory Fcγ receptor, such as one elected from the group consisting of FcγRI, FcγRIIa, FcγRIIIa, and mouse FcγRIV. Examples include human FcγRI, human FcγRIIa, human FcγRIIIa, mouse FcγRI, mouse FcγRIII, and mouse FcγRIV. As disclosed herein, based on such activating FcR binding affinity as well as inhibiting FcR binding affinity, one can calculate A/I ratios or select against agents or antibodies having high affinity for the activatory Fcγ receptor.

The above method can further include examining a fourth ability of the test agent to stimulate T cell responsiveness. The ability to stimulate can be dependent on an Fcγ receptor. To that end, the examining step can be carried out in an FcγRIIb humanized mouse and comprises measurement of T cell stimulation as compared to a native antibody. For example, one can identify an antibody as an adjuvant by testing the ability of the antibody to stimulate the immune system, wherein said testing involves substituting engagement of a receptor from the TNFR Superfamily (e.g. CD-40) and the natural ligand for the receptor from the TNFR Superfamily (e.g. CD-40L) with an agonistic antibody and the like. In certain aspects, the ability to stimulate is mediated by an Fcγ receptor (FcγR), in particular FcγRIIb and results in T-lymphocyte specific responsiveness. The above method can further include selecting the test agent as an adjuvant based on a measurable binding affinity of the test agent to the TNFR superfamily receptor or the inhibitory Fcγ receptor as compare to a control experiment in the absence of the test agent; thereby identifying the agent.

In a second aspect, the invention provides a method for making an agonistic antibody against a TNFR superfamily receptor. The method includes steps of providing a starting antibody or a first nucleic acid sequence encoding a polypeptide chain of the starting antibody; and modifying the starting antibody to obtain a modified antibody so that the modified antibody has a higher binding affinity to a TNFR superfamily receptor, or to an inhibitory Fcγ receptor, as compared to the starting antibody. The modifying step can be conducted by modifying the first nucleic acid sequence to obtain a second nucleic acid encoding a chain of the modified antibody. The modified antibody can exhibit an enhanced agonistic activity (including an enhanced adjuvant activity, an enhanced immunestimulatory activity, an enhanced apoptosis-inducing activity, and an enhanced ability to activate tumor specific T cell responses) as compared to the starting antibody. The TNFR superfamily receptor can be CD40 or DR5; the inhibitory Fcγ receptor can be human or mouse FcγRIIb.

In one embodiment, an Fc region of the modified antibody exhibits an increased binding affinity to FcγRIIb, as compared to the starting antibody. Via modifying binding affinity to human FcγRIIb (i.e., FcγRIIb-targeted Fc engineering), one can obtain modified antibodies that have desired abilities, including but not limited to, immunestimulatory and apoptosis-inducing activities or ability to activate tumor specific T cell responses. In one example, one can obtain mortified antibody with an Fc region that exhibits a decreased A/I ratio, as compared to the starting antibody. In that case, the modified antibody has an enhanced inhibitory binding affinity to FcγR and reduced ADCC as compared to the starting antibody. In another example, the modified antibody has an enhanced ability to stimulate the immune system of a mammalian animal and activating tumor specific T cell responses, wherein the ability to stimulate is mediated by an FcγR.

In a third aspect, the present invention also relates to an agonistic TNFR superfamily antibody identified, modified, or made according to the foregoing methods. In some embodiment, the antibody is able to stimulate the immune system of a mammalian animal and activating tumor specific T cell responses. In further aspects, the Fc region of the antibody is engineered to exhibit an increased binding affinity to FcγRIIb and/or a decreased A/I ratio, as compared to a parent or native sequence. In certain embodiments, such an agonistic antibody may exhibit an A/I ratio of less than 5.0, 4.0, 3.0, 2.0, 1.5, 1.0, or 0.9.

The Fc region of the foregoing antibody may comprise a IgG1 isotype (e.g. an amino acid sequence of any of SEQ ID NOS: 1 or 3) or a functional equivalent thereof having one or more amino acid substitutions adapted to improve FcγRIIb binding affinity and/or selectivity. Such amino acid substitutions may include, but are not limited to, an S267E amino acid substitution as provided in SEQ ID NO: 4. Alternatively, such amino acid substitutions may be selected from the group consisting of S267E, G236D, S239D, L328F I332E and combinations thereof.

Antibodies of the present invention may be provided as monoclonal antibodies or as engineered chimeric antibodies. In further embodiments, the antibody is an anti-CD40 or anti-DR5 antibody that is modified as provided above. Such an antibody may include, but is not limited to, αCD40:mIgG1, αCD40:hIgG1, αCD40:hIgG1(S267E), or αDR5:hIgG1(S267E) as disclosed herein.

In a fourth aspect, the invention provides a conjugate having a first segment that specifically binds to a TNFR superfamily receptor; and a second segment that binds to an inhibitory Fcγ receptor. The two segments can be linked covalently or non-covalently. The inhibitory Fcγ receptor can be human or mouse FcγRIIb. The TNFR superfamily receptor can be CD40 or DR5. In one example, the conjugate is an isolated fusion polypeptide and the two segments are heterologous to each other. For example, the first segment can contain the sequence of a CD40 ligand (e.g., CD154) or DR5 ligand (e.g., TNFSF10/TRAIL/APO-2L) and the second segment can include an Fc region of an antibody. In certain embodiments, there is a proviso that the conjugate is not a full-length or known anti-CD40 antibody, or is not a full-length or known anti-DR5 antibody.

The present invention further relates to pharmaceutical formulations containing therapeutically effective amounts of at least one or more of the antibodies or conjugates provided herein, which may include a pharmaceutically acceptable carrier. Such formulations may be administered to a subject to treat a targeted disease or disorder, such as a proliferative disease or disorder.

In further embodiments, the present invention further relates to methods of treating such a proliferative disease or disorder, comprising administering to a patient a therapeutically effective amount of an agonistic TNFR Superfamily antibody identified or made according to the foregoing or a conjugate described above. Such proliferative diseases include, but are not limited to, lymphoma, non-Hodgkins lymphoma (NHL), chronic lymphocytic leukemia (CLL), small lymphocytic lymphoma, leukemia, hairy cell leukemia (HCL), acute lymphocytic leukemia (ALL), and mantle cell lymphoma. Antibodies may be administered alone, in a formulations discussed herein and/or with one or more additional cytotoxic agents.

The invention further features use of the above-described agonistic antibodies, conjugates, or pharmaceutical formulation for treating a cellular proliferative disorder in a subject and in the manufacture of a medicament for treating a cellular proliferative disorder in a subject.

Additional embodiments and advantages will be readily apparent to one of skill in the art based on the disclosure provided herein.

Aspects and embodiments of the invention may also be provided in accordance with the following numbered paragraphs.
1. A method of identifying an agent useful as an adjuvant or an apoptotic agent, comprising
   obtaining a test agent;
   evaluating a first ability of the test agent to bind to a TNFR superfamily receptor; and
   evaluating a second ability of the test agent to bind to an inhibitory Fcγ receptor (FcγR), wherein the abilities of the test agent to bind both the TNFR superfamily receptor and the inhibitory FcγR indicate that the test agent is a candidate for an adjuvant or an apoptotic agent.
2. The method of paragraph 1, wherein the TNFR superfamily receptor contains one or more TNF receptor-associated factors (TRAF) interacting motifs and the test agent is a candidate for an adjuvant.
3. The method of paragraph 1, wherein the inhibitory Fcγ receptor is human or mouse FcγRIIb.
4. The method of paragraph 1, wherein the test agent comprises a polypeptide.
5. The method of paragraph 4, wherein the polypeptide comprises an Fc region of an antibody.
6. The method of paragraph 1, wherein the test agent is an antibody or a fusion polypeptide.
7. The method of paragraph 1, further comprising examining a third ability of the test agent to bind to an activatory Fcγ receptor.
8. The method of paragraph 7, wherein the activatory Fcγ receptor is selected from the group consisting of FcγRI, FcγRIIa, FcγRIIIa, and FcγRIV.
9. The method of paragraph 1, further comprising examining a fourth ability of the test agent to stimulate T cell responsiveness.
10. The method of paragraph 9, wherein said examining step is carried out in an FcγRIIb humanized mouse and comprises measurement of T cell stimulation as compared to a native antibody.
11. The method of paragraph 1, further comprising selecting the test agent as an adjuvant based on a measurable binding affinity of the test agent to the TNFR superfamily receptor or the inhibitory Fcγ receptor as compared to a control experiment in the absence of the test agent; thereby identifying the agent.
12. A method for making an agonistic antibody against a TNFR superfamily receptor, the method comprising:
   providing a starting antibody or a first nucleic acid sequence encoding a polypeptide chain of the starting antibody; and
   modifying the starting antibody to obtain a modified antibody so that the modified antibody has a higher binding affinity to an inhibitory Fcγ receptor, as compared to the starting antibody.
13. The method of paragraph 12, wherein the modified antibody exhibits an enhanced agonistic activity as compared to the starting antibody.
14. The method of paragraph 12, wherein the inhibitory Fcγ receptor is human or mouse FcγRIIb.
15. The method of paragraph 12, wherein an Fc region of the modified antibody exhibits an increased binding affinity to FcγRIIb, as compared to the starting antibody.
16. The method of paragraph 12, wherein an Fc region of the modified antibody exhibits a decreased A/I ratio, as compared to the starting antibody.
17. The method of paragraph 12, wherein the antibody has an ability to stimulate the immune system of a mammalian animal and activating tumor specific T cell responses, and said ability to stimulate is mediated by an Fcγ receptor (FcγR).
18. The method of paragraph 12, wherein the modified antibody has an enhanced inhibitory binding affinity to Fcγ receptors (FcγR) and reduced antibody-dependent cell-mediated cytotoxicity (ADCC) as compared to the starting antibody.
19. The method of paragraph 18, wherein the FcγR is human or mouse FcγRIIb.
20. The method of paragraph 12, wherein the modifying step is conducted by modifying the first nucleic acid sequence to obtain a second nucleic acid encoding a chain of the modified antibody.
21. An agonistic TNFR Superfamily antibody identified according to the method of any of paragraphs 1-11 or made according to the method of any of paragraphs 12-20.
22. The agonistic antibody of paragraph 21, wherein the antibody exhibits an A/I ratio of less than 5.0.
23. The agonistic antibody of paragraph 22, wherein the antibody exhibits an A/I ratio of less than 1.0.
24. The agonistic antibody of any of paragraphs 21-23, wherein the Fc region of the antibody comprises an amino acid sequence of any one of SEQ ID NO: 1, 77, or 3 or a functional equivalent thereof.
25. The agonistic antibody of paragraph 24, wherein the Fc region of the antibody comprises an amino acid substitution selected from the group consisting of S267E, G236D, S239D, L328F, I332E, and any combinations thereof.
26. The agonistic antibody of any of paragraphs 21-25, wherein the antibody is a monoclonal antibody.
27. The agonistic antibody of any of paragraphs 21-26, wherein the antibody is an engineered chimeric antibody.
28. The agonistic antibody of any of paragraphs 21-27, wherein the antibody is an agonistic anti- CD40 antibody or an agonistic anti-DR5 antibody.
29. The agonistic antibody of paragraph 28, wherein the antibody is αCD40:mIgG1, αCD40:hIgG1, or αCD40:hIgG1(S267E).
30. The agonistic antibody of paragraph 28, wherein the antibody is αDR5:hIgG1(S267E).
31. A conjugate, comprising
   a first segment that specifically binds to a TNFR superfamily receptor; and
   a second segment that binds to an inhibitory Fcγ receptor.
32. The conjugate of paragraph 31, wherein the inhibitory Fcγ receptor is human or mouse Fc FcγRIIb.
33. The conjugate of paragraph 31, wherein the TNFR superfamily receptor is CD40 or DR5.
34. The conjugate of paragraph 31, wherein the conjugate is an isolated fusion polypeptide and the two segments are heterologous to each other.
35. The conjugate of paragraph 34, wherein the first segment contains the sequence of a CD40 ligand or DR5 ligand.
36. The conjugate of paragraph 35, wherein the CD40 ligand is CD 154.
37. The conjugate of paragraph 35, wherein the DR5 ligand is TNFSF10/TRAIL/APO-2L.
38. The conjugate of paragraph 34, wherein the second segment comprises an Fc region of an antibody.
39. The conjugate of paragraph 31, with a proviso that said conjugate is not a full anti-CD40 antibody.
40. The conjugate of paragraph 31, with a proviso that said conjugate is not a full anti-DR5 antibody.
41. A pharmaceutical formulation comprising (i) an agonistic antibody of any of paragraphs 21- 30 or a conjugate of any of paragraphs 31-40 and (ii) a pharmaceutically acceptable carrier.
42. The pharmaceutical formulation of paragraph 41, wherein the agonistic antibody is a monoclonal antibody.
43. The pharmaceutical formulation of any of paragraphs 41 and 42, wherein the agonistic antibody is an anti-CD40 antibody or an anti-DR5 antibody.
44. The pharmaceutical formulation of any of paragraphs 41-43, wherein the antibody is an engineered chimeric anti-CD40 antibody or an anti-DR5 antibody.
45. The pharmaceutical formulation according to any of paragraphs 41-44, wherein the antibody is selected from the group consisting of αCD40:mIgG1, αCD40:hIgG1, ααCD40:hIgG1(S267E), and αDR5:hIgG1(S267E).
46. A method of treating a cellular proliferative disorder, comprising administering to a subject in need thereof a therapeutically effective amount of the agonistic antibody of any of paragraphs 21-29 or the conjugate of any of paragraphs 31-38.
47. The method of paragraph 46, wherein the agonistic antibody is a monoclonal antibody.
48. The method of any of paragraphs 46-47, wherein the antibody is an engineered chimeric antibody.
49. The method of any of paragraphs 46-49, wherein the proliferative disease or disorder is selected from the group consisting of lymphoma, non-Hodgkins lymphoma (NHL), chronic lymphocytic leukemia (CLL), small lymphocytic lymphoma, leukemia, hairy cell leukemia (HCL), acute lymphocytic leukemia (ALL), and mantle cell lymphoma.
50. The method of any of paragraphs 46-49, wherein the agonistic antibody is co-administered with a second cytotoxic agent.
51. Use of the agonistic antibody of any of paragraphs 21-30, the conjugate of any of paragraphs 31- 40, OΓ the pharmaceutical formulation of any of paragraphs 41-45 for treating a cellular proliferative disorder in a subject.
52. Use of the agonistic antibody of any of paragraphs 21-30, the conjugate of any of paragraphs 31- 40, or the pharmaceutical formulation of any of paragraphs 41-45 in the manufacture of a medicament for treating a cellular proliferative disorder in a subject.
53. The method of paragraph 2, wherein the TNFR superfamily receptor is CD40.
54. The method of paragraph 1, wherein the TNFR superfamily receptor contains one or more death domains and the test agent is a candidate for an apoptotic agent.
55. The method of paragraph 54, wherein the TNFR superfamily receptor is DR5.
56. The method of paragraph 12, wherein the modified antibody exhibits an enhanced apoptotic activity as compared to the starting antibody.
57. The method of paragraph 56, wherein the TNFR superfamily receptor is DR5.
58. The method of paragraph 12, wherein the modified antibody exhibits an enhanced adjuvant activity as compared to the starting antibody.
59. The method of paragraph 58, wherein the TNFR superfamily receptor is CD40.

### BRIEF DESCRIPTION OF THE DRAWINGS

FIGs. 1A and B illustrate that FcγRs are required for OVA-specific T cell response induced by DEC-hIgG1-OVA (OVA fused to an anti-DEC205 antibody with human IgG1 constant region) and anti-CD40 monoclonal antibody (αCD40mAb).
FIGs. 2A-D illustrate that a αCD40mAb without FcγR-binding capacity has no adjuvant effect.
FIGs. 3A-C illustrate that the adjuvant effect of αCD40mAb requires no activating FcγRs, but FcγRIIb.
FIGs. 4A-E illustrate that the adjuvant effect of αCD40mAb can be modulated by manipulating FcγR-binding.
FIG. 5 illustrates that αCD40mAb with enhanced human FcγRIIb binding has increased antitumor activity.
FIG. 6 illustrates that αCD40:mIgG1 is more protective than αCD40:mIgG2a in B6 B cell lymphoma (B6BL) model.
FIG. 7 illustrates that humanized αCD40mAbs with enhanced hFcγRIIb-binding is more protective in B6BL model and that the anti-tumor effect of αCD40:hIgG1(S267E) in B6BL model is human Fcγ RIIb trans gene-dependent.
FIGs. 8A-D illustrate that αCD40mAbs enhanced for FcγRIIb binding had greater antitumor activities than αCD40mAbs enhanced for activating FcγR binding in CD40⁺ tumor models (A, B, and D) and that the lack of anti-tumor activity for αCD40:mIgG2a was not due to defects in ADCC activity (C).
FIGs. 9A-C show that FcγRIIb is required for the liver damage and mortality induced by agonistic DR5 antibodies, where two biomarkers, serum aspartate transaminase (A) and alanine transaminase (B) were measured to assess liver damages, and survival curves (C) for two month were obtained.
FIGs. 10A and B show that FcγRIIb is required for the anti-tumor effects of agonistic DR5 antibodies, where wild-type C57BL/6 (A) and FcγRIIb-deficient (R2^{-/-}, B) mice were inoculated with 10⁶ MC38 cells subcutaneously, and treated with hamster IgG (hamIgG) or MD5-1 antibodies through intravenous injection, on days 7, 11, and 15.
FIGs. 11A and B show that the liver toxicity effect of agonistic DR5 antibodies can be enhanced by increasing human FcγRIIb-binding, where two biomarkers, serum aspartate transaminase (A) and alanine transaminase (B) were measured to assess liver damages.

### DETAILED DESCRIPTION OF THE INVENTION

The present invention relates, at least in part, to the unexpected discovery that antibodies engineered to have increased FcγRIIb binding affinity exhibit increased immunostimulatory activity and may be used as adjuvants and anti-tumor agents. The FcγRIIb receptor, as noted above, was originally viewed as an inhibitor of immunological stimulation due to previous observations that it decreased ADCC- and ADCP-based responsiveness and was required to maintain peripheral tolerance. Thus, mice made deficient in this receptor developed spontaneous autoimmune diseases, such as a lupus-like disease, resulting from their inability to maintain tolerance to self antigens. The data provided herein, however, surprisingly demonstrates that an agonistic antibody against a TNFR superfamily receptor (such as CD40 or DR5), engineered for increased FcγRIIb binding affinity, results in enhanced agonistic activities (including but not limited to, enhanced adjuvant activities, enhanced immunestimulatory and apoptosis-inducing activities, and enhanced activities to activate tumor specific T cell responses). Accordingly, the present invention is advantageous, *inter alia,* for identifying and developing agents (e.g., antibodies) with desired agonistic activities which may be used as a treatment method for diseases in which enhanced TNFR responsiveness is desirable.

### Definitions

To aid in the understanding of the invention, the following non-limiting definitions are provided:
Throughout the present specification and claims, the numbering of the residues in an immunoglobulin heavy chain is that of the EU index as in Kabat et al., Sequences of Proteins of Immunological Interest, 5th Ed. Public Health Service, National Institutes of Health, Bethesda, Md. (1991), which is expressly incorporated herein by reference. The "EU index as in Kabat" refers to the residue numbering of the human IgG1 EU antibody.

The term "native," "parent," or "starting" refers to an antibody comprising an amino acid sequence which lacks one or more of the Fc region modifications disclosed herein and which differs in effector function compared to a modified antibody as herein disclosed. The parent polypeptide may comprise a native sequence Fc region or an Fc region with pre-existing amino acid sequence modifications (such as additions, deletions and/or substitutions).

The term "Fc region" is used to define a C-terminal region of an immunoglobulin heavy chain. The "Fc region" may be a native sequence Fc region or a variant Fc region. The boundaries of the Fc region of an immunoglobulin heavy chain might vary. In some references, the human IgG heavy chain Fc region is defined to stretch from an amino acid residue at position Cys226, or from Pro230, to the carboxyl-terminus thereof. For the purposes of this invention, the term is defined as starting at amino acid 210 (as in Kabat) and ending at the carboxy terminus of the heavy chain.

A "native sequence Fc region" comprises an amino acid sequence identical to the amino acid sequence of an Fc region found in nature. A "variant Fc region" comprises an amino acid sequence which differs from that of a native sequence Fc region by virtue of at least one "amino acid modification" as herein defined. Preferably, the variant Fc region has at least one amino acid substitution compared to a native sequence Fc region or to the Fc region of a parent polypeptide, e.g., from about one to about ten amino acid substitutions, and preferably from about one to about five amino acid substitutions in a native sequence Fc region or in the Fc region of the parent polypeptide. Outside of the mutations specified herein, the variant Fc region herein will preferably possess at least about 80% homology with a native sequence Fc region and/or with an Fc region of a parent polypeptide, and most preferably at least about 90% homology therewith, more preferably at least about 95% homology therewith, even more preferably, at least about 99% homology therewith, or most preferably, 100% homology therewith.

The terms "Fc receptor" or "FcR" are used to describe a receptor that binds to the Fc region of an antibody. The preferred FcR is a native sequence human FcR. Moreover, a preferred human FcR is one which binds an IgG antibody (a gamma receptor) and includes receptors of the human FcγRI, FcγRII, and FcγRIII subclasses, including allelic variants and alternatively spliced forms of these receptors. FcγRII receptors include FcγRIIa (an "activating receptor") and FcγRIIb (an "inhibiting receptor"), which have similar amino acid sequences that differ primarily in the cytoplasmic domains thereof. Activating receptor FcγRIIa contains an immunoreceptor tyrosine-based activation motif (ITAM) in its cytoplasmic domain. Inhibiting receptor FcγRIIb contains an immunoreceptor tyrosine-based inhibition motif (ITIM) in its cytoplasmic domain (see Daron, Annu Rev Immunol, 15, 203-234 (1997); FcRs are reviewed in Ravetch and Kinet, Annu Rev Immunol, 9, 457-92 (1991); Capel et al., Immunomethods, 4, 25-34 (1994); and de Haas et al., J Lab Clin Med, 126, 330-41 (1995), each of which is incorporated herein by reference).

The term "epitope" refers to the region of an antigen to which an antibody or T cell binds.

An "antigen" refers to a substance that elicits an immunological reaction or binds to the products of that reaction.

"Antibody-dependent cell-mediated cytotoxicity" and "ADCC" refer to an in vitro or in vivo cell-mediated reaction in which nonspecific cytotoxic cells that express FcRs (e.g., monocytic cells such as natural killer (NK) cells and macrophages) recognize bound antibody on a target cell and subsequently cause lysis of the target cell. In principle, any effector cell with an activating FcγR can be triggered to mediate ADCC. One such cell the NK cell, express FcγRIII only, whereas monocytes, depending on their state of activation, localization, or differentiation, can express FcγRI, FcγRII, and FcγRIII. FcR expression on hematopoietic cells is summarized in Ravetch and Bolland, Annu Rev Immunol, (2001), which is incorporated herein by reference.

The term "antibody" is used in the broadest sense and specifically covers monoclonal antibodies (including full length monoclonal antibodies), polyclonal antibodies, multispecific antibodies (e.g., bispecific antibodies), and antibody fragments so long as they exhibit the desired biological activity.

The term "monoclonal antibody" as used herein refers to an antibody obtained from a population of substantially homogeneous antibodies, i.e., the individual antibodies comprising the population are identical except for possible naturally occurring mutations that may be present in minor amounts. Monoclonal antibodies are highly specific, being directed against a single antigenic site. Furthermore, in contrast to conventional (polyclonal) antibody preparations that typically include different antibodies directed against different determinants (epitopes), each monoclonal antibody is directed against a single determinant on the antigen. The modifier "monoclonal" indicates the character of the antibody as being obtained from a substantially homogeneous population of antibodies, and is not to be construed as requiring production of the antibody by any particular method. For example, the monoclonal antibodies to be used in accordance with the present invention may be made by the hybridoma method first described by Kohler and Milstein, Nature, 256, 495-497 (1975), which is incorporated herein by reference, or may be made by recombinant DNA methods (see, e.g., U.S. Patent No. 4,816,567, which is incorporated herein by reference). The monoclonal antibodies may also be isolated from phage antibody libraries using the techniques described in Clackson et al., Nature, 352, 624-628 (1991) and Marks et al., J Mol Biol, 222, 581-597 (1991), for example, each of which is incorporated herein by reference. Monoclonal antibodies can be isolated from transgenic animals

The monoclonal antibodies herein specifically include "chimeric" antibodies (immunoglobulins) in which a portion of the heavy and/or light chain is identical with or homologous to corresponding sequences in antibodies derived from a particular species or belonging to a particular antibody class or subclass, while the remainder of the chain(s) is identical with or homologous to corresponding sequences in antibodies derived from another species or belonging to another antibody class or subclass, as well as fragments of such antibodies, so long as they exhibit the desired biological activity (see U.S. Patent No. 4,816,567; Morrison et al., Proc Natl Acad Sci USA, 81, 6851-6855 (1984); Neuberger et al., Nature, 312, 604-608 (1984); Takeda et al., Nature, 314, 452-454 (1985); International Patent Application No. PCT/GB85/00392, each of which is incorporated herein by reference).

"Humanized" forms of non-human (e.g., murine) antibodies are chimeric antibodies that contain minimal sequence derived from non-human immunoglobulin. For the most part, humanized antibodies are human immunoglobulins (recipient antibody) in which residues from a hypervariable region of the recipient are replaced by residues from a hypervariable region of a non-human species (donor antibody) such as mouse, rat, rabbit or nonhuman primate having the desired specificity, affinity, and capacity. In some instances, Fv framework region (FR) residues of the human immunoglobulin are replaced by corresponding non-human residues. Furthermore, humanized antibodies may comprise residues that are not found in the recipient antibody or in the donor antibody. These modifications are made to further refine antibody performance. In general, the humanized antibody will comprise substantially all of at least one, and typically two, variable domains, in which all or substantially all of the hypervariable loops correspond to those of a non-human immunoglobulin and all or substantially all of the FR residues are those of a human immunoglobulin sequence. The humanized antibody optionally also will comprise at least a portion of an immunoglobulin constant region (Fc), typically that of a human immunoglobulin. For further details, see Jones et al., Nature, 321, 522-525 (1986); Riechmann et al., Nature, 332, 323-329 (1988); Presta, Curr Op Struct Biol, 2, 593-596 (1992); U.S. Patent No. 5,225,539, each of which is incorporated herein by reference.

Human antibodies refer to any antibody with fully human sequences, such as might be obtained from a human hybridoma, human phage display library or transgenic mouse expressing human antibody sequences.

The term "about" refers to a range of values which would not be considered by a person of ordinary skill in the art as substantially different from the baseline values. When this term is used in conjunction to binding affinity to Fc receptors, it refers to a range between 5-25% of the baseline values. When this term refers to the homology and/or similarity of the amino acid sequences, this term refers to the range within 10% of the baseline value.

A "chimeric" or "fusion" refers to the combination of amino acid sequences of different origin in one polypeptide chain by in-frame combination of their coding nucleotide sequences. The term explicitly encompasses internal fusions, i.e., insertion of sequences of different origin within a polypeptide chain, in addition to fusion to one of its termini.

A heterologous protein, polypeptide, nucleic acid, or gene is one that originates from a foreign species, or, if from the same species, is substantially modified from its original form. Two fused domains or sequences are heterologous to each other if they are not adjacent to each other in a naturally occurring protein or nucleic acid.

The terms "peptide," "polypeptide," and "protein" are used herein interchangeably to describe the arrangement of amino acid residues in a polymer. A peptide, polypeptide, or protein can be composed of the standard 20 naturally occurring amino acid, in addition to rare amino acids and synthetic amino acid analogs. They can be any chain of amino acids, regardless of length or post-translational modification (for example, glycosylation or phosphorylation). The peptide, polypeptide, or protein "of this invention" includes recombinantly or synthetically produced fusion versions having the particular domains or portions of a TNFR superfamily receptor or an inhibitory Fcγ receptor. The term also encompasses polypeptides that have an added amino-terminal methionine (useful for expression in prokaryotic cells).

An "isolated" or "purified" peptide, polypeptide, or protein refers to a peptide, polypeptide, or protein that has been separated from other proteins, lipids, and nucleic acids with which it is naturally associated. The polypeptide/protein can constitute at least 10% (i.e., any percentage between 10% and 100%, e.g., 20%, 30%, 40%, 50%, 60%, 70 %, 80%, 85%, 90%, 95%, and 99%) by dry weight of the purified preparation. Purity can be measured by any appropriate standard method, for example, by column chromatography, polyacrylamide gel electrophoresis, or HPLC analysis. An isolated polypeptide/protein described in the invention can be purified from a natural source, produced by recombinant DNA techniques, or by chemical methods.

As used herein, a "subject" refers to a human and a non-human animal. Examples of a non-human animal include all vertebrates, e.g., mammals, such as non-human mammals, non-human primates (particularly higher primates), dog, rodent (e.g., mouse or rat), guinea pig, cat, and rabbit, and non-mammals, such as birds, amphibians, reptiles, etc. In one embodiment, the subject is a human. In another embodiment, the subject is an experimental, non-human animal or animal suitable as a disease model.

As used herein, "treating" or "treatment" refers to administration of a compound or agent to a subject who has a disorder with the purpose to cure, alleviate, relieve, remedy, delay the onset of, prevent, or ameliorate the disorder, the symptom of the disorder, the disease state secondary to the disorder, or the predisposition toward the disorder.

An "effective amount" or "therapeutically effective amount" refers to an amount of the compound or agent that is capable of producing a medically desirable result in a treated subject. The treatment method can be performed *in vivo* or *ex vivo,* alone or in conjunction with other drugs or therapy. A therapeutically effective amount can be administered in one or more administrations, applications or dosages and is not intended to be limited to a particular formulation or administration route.

The agent can be administered *in vivo* or *ex vivo,* alone or co-administered in conjunction with other drugs or therapy, i.e., a cocktail therapy. As used herein, the term "co-administration" or "co-administered" refers to the administration of at least two agents or therapies to a subject. In some embodiments, the co-administration of two or more agents/therapies is concurrent. In other embodiments, a first agent/therapy is administered prior to a second agent/therapy. Those of skill in the art understand that the formulations and/or routes of administration of the various agents/therapies used may vary.

As used herein, the term "adjuvant agent" or "adjuvant" means a substance added to an immunogenic composition or a vaccine to increase the immunogenic composition or the vaccine's immunogenicity. An apoptotic agent or pro-apoptotic agent refers to any agent that induces apoptosis.

The terms "agonist" and "agonistic" when used herein refer to or describe a molecule which is capable of, directly or indirectly, substantially inducing, promoting or enhancing, biological activity or activation of a TNFR superfamily receptor. Optionally, an agonistic anti-CD40 or anti-DR5 antibody is an antibody which has activity that mimics or is comparable to CD40 or DR5 ligand. Preferably, the agonist is a molecule which is capable of inducing immunestimulation and apoptosis in a mammalian cell. Even more preferably, the agonist is an antibody directed to a TNFR superfamily receptor and said antibody has the activity which is equal to or greater than the 1C10 anti-CD40 antibody or the MD5-1 anti-DR5 antibody described in the Examples below. Optionally, the agonist activity of such molecule can be determined by assaying the molecule, alone or in a cross-linked form using Fc immunoglobulin or complement (described below), in an assay described in the examples to examine immunestimulation and/or apoptosis of cells or other cells which express a TNFR superfamily receptor, such as CD40 or DR5.

The term "cytotoxic agent" as used herein refers to a substance that inhibits or prevents the function of cells and/or causes destruction of cells. The term is intended to include radioactive isotopes (e.g. At²¹¹, I¹³¹, I¹²⁵, Y⁹⁰, Re¹⁸⁶, Re¹⁸⁸, Sm¹⁵³, Bi²¹², P³² and radioactive isotopes of Lu), chemotherapeutic agents, and toxins such as small molecule toxins or enzymatically active toxins of bacterial, fungal, plant or animal origin, including fragments and/or variants thereof.

A "chemotherapeutic agent" is a chemical compound useful in the treatment of cancer. Examples of chemotherapeutic agents include alkylating agents such as thiotepa and cyclosphosphamide (CYTOXAN™); alkyl sulfonates such as busulfan, improsulfan and piposulfan; aziridines such as benzodopa, carboquone, meturedopa, and uredopa; ethylenimines and methylamelamines including altretamine, triethylenemelamine, trietylenephosphoramide, triethylenethiophosphaoramide and trimethylolomelamine; acetogenins (especially bullatacin and bullatacinone); a camptothecin (including the synthetic analogue topotecan); bryostatin; callystatin; CC-1065 (including its adozelesin, carzelesin and bizelesin synthetic analogues); cryptophycins (particularly cryptophycin 1 and cryptophycin 8); dolastatin; duocarmycin (including the synthetic analogues, KW-2189 and CBI-TMI); eleutherobin; pancratistatin; a sarcodictyin; spongistatin; nitrogen mustards such as chlorambucil, chlornaphazine, cholophosphamide, estramustine, ifosfamide, mechlorethamine, mechlorethamine oxide hydrochloride, melphalan, novembichin, phenesterine, prednimustine, trofosfamide, uracil mustard; nitrosureas such as carmustine, chlorozotocin, fotemustine, lomustine, nimustine, ranimustine; antibiotics such as the enediyne antibiotics (e.g. calicheamicin, see, e.g., Agnew Chem. Intl. Ed. Engl. 33:183-186 (1994); dynemicin, including dynemicin A; an esperamicin; as well as neocarzinostatin chromophore and related chromoprotein enediyne antibiotic chromomophores), aclacinomysins, actinomycin, authramycin, azaserine, bleomycins, cactinomycin, carabicin, caminomycin, carzinophilin, chromomycins, dactinomycin, daunorubicin, detorubicin, 6-diazo-5-oxo-L-norleucine, doxorubicin (including morpholino-doxorubicin, cyanomorpholino-doxorubicin, 2-pyrrolino-doxorubicin and deoxydoxorubicin), epirubicin, esorubicin, idarubicin, marcellomycin, mitomycins, mycophenolic acid, nogalamycin, olivomycins, peplomycin, potfiromycin, puromycin, quelamycin, rodorubicin, streptonigrin, streptozocin, tubercidin, ubenimex, zinostatin, zorubicin; anti-metabolites such as methotrexate and 5-fluorouracil (5-FU); folic acid analogues such as denopterin, methotrexate, pteropterin, trimetrexate; purine analogs such as fludarabine, 6-mercaptopurine, thiamiprine, thioguanine; pyrimidine analogs such as ancitabine, azacitidine, 6-azauridine, carmofur, cytarabine, dideoxyuridine, doxifluridine, enocitabine, floxuridine, 5-FU; androgens such as calusterone, dromostanolone propionate, epitiostanol, mepitiostane, testolactone; anti-adrenals such as aminoglutethimide, mitotane, trilostane; folic acid replenisher such as frolinic acid; aceglatone; aldophosphamide glycoside; aminolevulinic acid; amsacrine; bestrabucil; bisantrene; edatraxate; defofamine; demecolcine; diaziquone; elformithine; elliptinium acetate; an epothilone; etoglucid; gallium nitrate; hydroxyurea; lentinan; lonidamine; maytansinoids such as maytansine and ansamitocins; mitoguazone; mitoxantrone; mopidamol; nitracrine; pentostatin; phenamet; pirarubicin; podophyllinic acid; 2-ethylhydrazide; procarbazine; PSK®.; razoxane; rhizoxin; sizofuran; spirogermanium; tenuazonic acid; triaziquone; 2,2',2"-trichlorotriethylamine; trichothecenes (especially T-2 toxin, verracurin A, roridin A and anguidine); urethan; vindesine; dacarbazine; mannomustine; mitobronitol; mitolactol; pipobroman; gacytosine; arabinoside ("Ara-C"); cyclophosphamide; thiotepa; taxoids, e.g. paclitaxel (TAXOL®, Bristol-Myers Squibb Oncology, Princeton, N.J.) and doxetaxel (TAXOTERE®, Rhone-Poulenc Rorer, Antony, France); chlorambucil; gemcitabine; 6-thioguanine; mercaptopurine; methotrexate; platinum analogs such as cisplatin and carboplatin; vinblastine; platinum; etoposide (VP-16); ifosfamide; mitomycin C; mitoxantrone; vincristine; vinorelbine; navelbine; novantrone; teniposide; daunomycin; aminopterin; xeloda; ibandronate; CPT-11; topoisomerase inhibitor RFS 2000; difluoromethylornithine (DMFO); retinoic acid; capecitabine; and pharmaceutically acceptable salts, acids or derivatives of any of the above. Also included in this definition are anti-hormonal agents that act to regulate or inhibit hormone action on tumors such as antiestrogens including for example tamoxifen, raloxifene, aromatase inhibiting 4(5)-imidazoles, 4-hydroxytamoxifen, trioxifene, keoxifene, LY117018, onapristone, and toremifene (Fareston); and anti-androgens such as flutamide, nilutamide, bicalutamide, leuprolide, and goserelin; and pharmaceutically acceptable salts, acids or derivatives of any of the above.

In one aspect, the present invention relates to methods of identifying, modifying, or making an antibody as an adjuvant using standard molecular techniques and evaluating or testing the ability of the antibody to stimulate a T-lymphocyte response. Variant Fc portions of such antibodies are specifically engineered or modified from a parent or native sequence Fc region to have higher binding affinities to FcγRIIb receptors. In certain embodiments, the antibodies also exhibit selective binding affinity to FcγRIIb over other Fc receptors, particularly those thought to elicit non-specific immunological responsiveness (e.g. FcyRI, FcyRIIa/c, and FcγRIIIa). To this end, the antibodies of the present invention result in T-lymphocyte specific responsiveness, while minimizing the stimulation of relatively non-specific ADCC and/or ADCP pathways.

Adjuvant activity for certain agonistic TNFR antibodies can be predicted based upon the binding affinity and selectivity to the FcγRIIb receptor. Accordingly, in one aspect, the present invention relates to a method for determining such activity by: (a) determining a binding affinity of the antibody to activating Fc receptors (e.g. FcγRI, FcγRIIa/c, and FcγRIIIa) or other receptors considered to activating in a ADCC or ADCP context; (b) determining a binding affinity of the antibody to inhibitory Fc receptors (e.g. FcyRIIb) or other receptors considered to inhibitory in a ADCC or ADCP context, and (c) calculating the A/I ratio (i.e. ratio of (a):(b)) of said binding affinities.

The A/I ratio serves a predictor of the *in vivo* activity of an antibody or variant wherein the magnitude of said ratio is an indication its adjuvant activity. One aspect of the present invention provides a general and widely applicable method of selecting an adjuvant antibody or variant thereof out of a plurality of antibodies comprising: comparing the A/I ratios of the plurality of antibodies; and selecting the antibody or variant thereof with an A/I ratio less than one (i.e. greater binding affinity to FcγRIIb). The identified Fc region may then be paired with an antigen binding domain, such as a Fab, to a targeted receptor defined herein or otherwise known in the art. In one embodiment the antibody or variant thereof has an A/I ratio of between 0.1 and 0.9. In another embodiment the antibody or variant thereof has an A/I ratio of between 0.2 and 0.7.

An A/I ratio may be determined in the manner as described in WO 2007/055916 and US Application No. 20080286819, the contents which are incorporated by reference. A person who practices the method of the instant invention should keep in mind that the activating Fcγ receptor subtype should, preferably, be considered in determining the appropriate A/I ratio and, thus, the selection of the appropriate antibody. It is preferred that the A/I ratio should be calculated using the binding data for a receptor through which an antibody isotype exerts its effect. By way of example only and without any limitation, a person of the ordinary skill in the art will appreciate that if the FcγRIIIa/FcγRIIb ratio is higher than the Fcγ.RIIa/FcγRIIb ratio this antibody isotype will exert its effect via the FcγRIIIa receptor and not the FcγRIIa receptor. Accordingly, the binding data for FcγRIIIa would be used for determining the appropriate A/I ratio.

The A/I ratio may be determined using one or more standard quantitative assays generally known in the art including those described in WO 2007/055916 and US Application No. 20080286819, the contents which are incorporated by reference. Such assays may include, but are not limited to, competition or sandwich ELISA, a radioimmunoassay, a dot blot assay, a fluorescence polarization assay, a scintillation proximity assay, a homogeneous time resolved fluorescence assay, a resonant mirror biosensor analysis, and a surface plasmon resonance analysis. In the competition or sandwich ELISA, the radioimmunoassay, or the dot blot assay the A/I ratio can be determined by coupling the assay with a statistical analysis method, such as, for example, Scatchard analysis. Scatchard analysis is widely known and accepted in the art and is described in, for example, Munson et al., Anal Biochem, 107:220 (1980), the contents of which are incorporated herein by reference.

In certain embodiments, the Fc region of the antibody comprises or is a variant of a human IgG1, IgG2, IgG3 or IgG4 region, wherein the Fc region is modified or engineered from the parent or native sequence to exhibit improved binding and/or selectivity to FcyRIIb, i.e. a lower A/I ratio than the parent or native sequence Fc region. Such antibodies may be either derived from a naturally occurring antibody or expressed in a cell line. In one embodiment, the Fc region includes a modification of the hIgG1 amino acid sequence. While not limited thereto, the hIgG1 heavy chain, IgG1 light chain, and IgG1 Fc region are provided in SEQ ID NOs: 1-3, 77, and 78, as follows and with the leader sequence being underlined:
αCD40:hIgG1 heavy chain sequence including VH and human IgG1 constant region: 
MDIRLSLVFLVLFIKGVQCEVQLVESDGGLVQPGRSLKLPCAASGFTFSDYYMAWVRQAPTKGL
EWVASISYDGSSTYYRDSVKGRFTISRDNAKSTLYLQMDSLRSEDTATYYCGRHSSYFDYWGQG
VMVTVSSASTKGPSVFPLAPSSKSTSGGTAALGCLVKDYFPEPVTVSWNSGALTSGVHTFPAVL
QSSGLYSLSSVVTVPSSSLGTQTYICNVNHKPSNTKVDKRVEPKSCDKTHTCPPCPAPELLGGP
SVFLFPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQVSLT
CLVKGFYPSDIAVEWESNGQPENNYKTTPPVLDSDGSFFLYSKLTVDKSRWQQGNVFSCSVMHE
ALHNHYTQKSLSLSPGK (SEQ ID NO: 1)
αCD40:hIgG1 light chain sequence including VL and human kappa constant region: 
METDRLLLWVLLLWVPGSTGDTVLTQSPALAVSPGERVTISCRASDSVSTLMHWYQQKPGQQPK
LLIYLASHLESGVPARFSGSGSGTDFTLTIDPVEADDTATYYCQQSWNDPWTFGGGTKLELKRT
VAAPSVFIFPPSDEQLKSGTASVVCLLNNFYPREAKVQWKVDNALQSGNSQESVTEQDSKDSTY
SLSSTLTLSKADYEKHKVYACEVTHQGLSSPVTKSFNRGEC (SEQ ID NO: 2)
Fc of αCD40(hIgG1) (starting from amino acid 210 in Kabat system): 
KVDKRVEPKSCDKTHTCPPCPAPELLGGPSVFLFPPKPKDTLMISRTPEVTCVVVDVSHEDPEV
KFNWYVDGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK (SEQ ID NO: 3)
αDR5 (hIgG1) HC sequences (Leader exon is underlined): 






 

 (SEQ ID NO: 77)
αDR5 (hIgG1) LC sequences (Leader exon is underlined):

MAMKVPAQALVILLLWVSGATCDIQVTQSPLLSASFGDKVTINCLVTQDITYYLSWYQQKSGQ
PPTLLIYNGNSLQSGVPSRFSGQYSGRTFTLSLSSLEPEDAGTYYCLQHYSVPFTFGGGTRLEI
KRTVAAPSVFIFPPSDEQLKSGTASVVCLLNNFYPREAKVQWKVDNALQSGNSQESVTEQDSKD
STYSLSSTLTLSKADYEKHKVYACEVTHQGLSSPVTKSFNRGEC (SEQ ID NO: 78)

Such modifications may be at one or more positions, which result in higher and/or more selective FcγRIIb binding. Such modifications may include, for example, amino acid substitutions corresponding to positions 267 of the Fc portion. In further embodiments, the Serine at position 267 is changed to include a Glutamate, as follows:
Fc of αCD40:hIgG1(S267E) (S267E is underlined):

KVDKRVEPKSCDKTHTCPPCPAPELLGGPSVFLFPPKPKDTLMISRTPEVTCVVVDVEHEDPEV
KFNWYVDGVEVHNAKTKPREEQYNSTYRVVSVLTVLHQDWLNGKEYKCKVSNKALPAPIEKTIS
KAKGQPREPQVYTLPPSRDELTKNQVSLTCLVKGFYPSDIAVEWESNGQPENNYKTTPPVLDSD
GSFFLYSKLTVDKSRWQQGNVFSCSVMHEALHNHYTQKSLSLSPGK (SEQ ID NO: 4)

In alternative embodiments, the IgG1 may include one or a combination of G236D/S267E, S239D/S267E, and/or S267E/L328F, as disclosed within Chu, et al. Mol. Immunol. 2008 Sep;45(15):3926-33, the contents of which are incorporated herein by reference.

While the order of steps is not necessarily limiting to the invention, the foregoing Fc region(s) are paired with an antigen binding domain (e.g. Fab region) specifically engineered with one or more CDRs to a targeted epitope, such as, but not limited to, a cell surface receptor. In certain aspects, the antigen binding domain of the antibody agonistically engages a receptor from the TNFR Superfamily and its natural ligand. Receptors and ligands of the TNFR Superfamily include, but are not limited to, one or a combination of the following: CD120 (including isoforms CD120a and CD120b), Lymphotoxin β receptor, CD134, CD40, FAS, TNFRSF6B, CD27, CD30, CD137, TNFRSF10 (including isoforms TNFRSF10A, TNFRSF10B, TNFRSF10C, and TNFRSF10D), RANK, Osteoprotegerin, TNFRSF12A, TNFRSF13B, TNFRSF13C, TNFRSF14, Nerve growth factor receptor, TNFRSF17, TNFRSF18, TNFRSF19, TNFRSF21, TNFRSF25, and Ectodysplasin A2 receptor. Antigen binding domains containing one or a combination of these targets may be developed using one or a combination of techniques commonly known in the art or otherwise discussed herein. The general structure and properties of CDRs within naturally occurring antibodies have been described in the art. Briefly, in a traditional antibody scaffold, the CDRs are embedded within a framework in the heavy and light chain variable region where they constitute the regions largely responsible for antigen binding and recognition. A variable region comprises at least three heavy or light chain CDRs (Kabat et al., 1991, Sequences of Proteins of Immunological Interest, Public Health Service N.I.H., Bethesda, Md.; see also Chothia and Lesk, 1987, J. Mol. Biol. 196:901-917; Chothia et al., 1989, Nature 342: 877-883), within a framework region (designated framework regions 1-4, FR1, FR2, FR3, and FR4, by Kabat *et al.,* 1991; see also Chothia and Lesk, 1987, supra). The CDRs provided by the present invention, however, may not only be used to define the antigen binding domain of a traditional antibody structure, but may be embedded in a variety of other scaffold structures, as generally understood in the art.

Based on the foregoing, and in certain non-limiting embodiments, the TNFR Superfamily includes the CD40 receptor, and the antibody is an agonistic anti-CD40 antibody. To this end, the agonistic anti-CD40 antibody may include CDR regions that are adapted to bind to an epitope on the CD-40 receptor that mimics engagement of its natural ligand CD40L. Such CDR regions may include, but are not limited to, one or a combination of the following:

| | |
|---|---|
| αCD40_VH_CDR1: DYYMA | (SEQ ID NO: 5) |
| αCD40_VH_CDR2: SISYDGSSTYYRDSVKG | (SEQ ID NO: 6) |
| αCD40_VH_CDR3: HSSYFDY | (SEQ ID NO: 7) |
| αCD40_VL_CDR1: RASDSVSTLMH | (SEQ ID NO: 8) |
| αCD40_VL_CDR2: LASHLES | (SEQ ID NO: 9) |
| αCD40_VL_CDR3: QQSWNDPWT | (SEQ ID NO: 10) |

In other embodiments, the TNFR Superfamily includes the DR5 receptor, and the antibody is an agonistic anti-DR5 antibody. In that case, the agonistic anti-DR5 antibody can include CDR regions that are adapted to bind to an epitope on the DR5 receptor that mimics engagement of its natural ligand TNFSF10/TRAIL/APO-2L. Such CDR regions may include, but are not limited to, one or a combination of the following:

| | |
|---|---|
| αDR5_VH_CDR1: AGGYWWT | (SEQ ID NO: 79) |
| αDR5_VH_CDR2: YIYSSGSTNYNPSIKS | (SEQ ID NO: 80) |
| αDR5_VH_CDR3: AGTSYSGFFDS | (SEQ ID NO: 81) |
| αDR5_VL_CDR1: LVTQDITYYLS | (SEQ ID NO: 82) |
| αDR5_VL_CDR2: NGNSLQS | (SEQ ID NO: 83) |
| αDR5_VL_CDR3: LQHYSVPFT | (SEQ ID NO: 84) |

The agonistic anti-CD40 or anti-DR5 antibody also includes a modified Fc region exhibiting improved or selective FcRIIb affinity, such as, but not limited to, one or a combination of the IgG1 amino acid substitutions provided above.

Antibodies or portions of antibodies, to include the Fc and Fab regions, of the present invention may be manufactured or developed as monoclonal antibodies, murine antibodies, human antibodies, chimeric antibodies, or humanized antibodies, as defined herein or otherwise known in the art. Such antibodies, or portions thereof, may be manufactured using standard techniques known in the art. To this end, modified antibodies in accordance with the foregoing include those in which specific amino acid substitutions, additions or deletions are introduced into a parental sequence through the use of recombinant DNA techniques to modify the genes encoding the heavy chain constant region. The introduction of these modifications follows well-established techniques of molecular biology, as described in manuals such as Molecular Cloning (Sambrook and Russel, (2001)), the contents of which are incorporated herein by reference. In addition, modified antibodies will include those antibodies which have been selected to contain specific carbohydrate modifications, obtained either by expression in cell lines known for their glycosylation specificity (Stanley P., et al., Glycobiology, 6, 695-9 (1996); Weikert S., et al., Nature Biotechnology, 17, 1116-1121 (1999); Andresen DC and Krummen L., Current Opinion in Biotechnology, 13, 117-123 (2002), the contents each of which are incorporated herein by reference.) or by enrichment or depletion on specific lectins or by enzymatic treatment (Hirabayashi et al., J Chromatogr B Analyt Technol Biomed Life Sci, 771, 67-87 (2002); Robertson and Kennedy, Bioseparation, 6, 1-15 (1996), the contents each of which are incorporated herein by reference.). It is known in the art that quality and extent of antibody glycosylation will differ depending on the cell type and culture condition employed. See, for example, Patel et al., Biochem J, 285, 839-845 (1992) and Kunkel et al., Biotechnol Prog, 16, 462-470 (2000), the contents of which are incorporated herein by reference.

One or a combination of the foregoing antibodies may be used for treatment of a disease or disorder, particularly, though not exclusively, a cellular proliferative disease or disorder. A cellular proliferative disorder refers to a disorder characterized by uncontrolled, autonomous cell growth, including non-malignant and malignant growth disorder, such as cancer or neoplastic diseases. Examples of the cellular proliferative disorder include pancreatic cancer, colon cancer, breast cancer, prostate cancer, hepatocellular carcinoma, melanoma, lung cancer, glioblastoma, brain tumor, hematopoietic malignancies, retinoblastoma, renal cell carcinoma, head and neck cancer, cervical cancer, esophageal cancer, and squamous cell carcinoma. Additional examples of such diseases include, but are not limited to, lymphoma, non-Hodgkins lymphoma (NHL), chronic lymphocytic leukemia (CLL), small lymphocytic lymphoma, leukemia, hairy cell leukemia (HCL), acute lymphocytic leukemia (ALL), and mantle cell lymphoma. In accordance with the foregoing, such antibodies are specifically adapted to stimulate the immune system of a mammalian animal and activate a tumor specific T cell response. Such antibodies may be provided in any effective amount or therapeutically effective amount that is sufficient to treat the targeted disease or disorder.

Such antibodies may be provided or otherwise delivered with optional pharmaceutically acceptable carriers, excipients or stabilizers (see, e.g., Remington's Pharmaceutical Sciences 16th edition, Osol, A. Ed. (1980), the contents of which are incorporated herein by reference), in the form of lyophilized formulations or aqueous solutions. Acceptable carriers, excipients, or stabilizers are nontoxic to recipients at the dosages and concentrations employed, and include buffers such as phosphate, citrate, and other organic acids; antioxidants including ascorbic acid and methionine; preservatives (such as octadecyldimethylbenzyl ammonium chloride; hexamethonium chloride; benzalkonium chloride, benzethonium chloride; phenyl, butyl or benzyl alcohol; alkyl parabens such as methyl or propyl paraben; catechol; resorcinol; cyclohexanol; 3-pentanol; and m-cresol); low molecular weight (less than about 10 residues) polypeptide; proteins, such as serum albumin, gelatin, or immunoglobulins; hydrophilic polymers such as polyvinylpyrrolidone; amino acids such as glycine, glutamine, asparagine, histidine, arginine, or lysine; monosaccharides, disaccharides, and other carbohydrates including glucose, mannose, or dextrins; chelating agents such as EDTA; sugars such as sucrose, mannitol, trehalose or sorbitol; salt-forming counter-ions such as sodium; metal complexes (e.g., Zn-protein complexes); and/or non-ionic surfactants such as TWEEN™, PLURONICS™ or polyethylene glycol (PEG).

The formulations herein may also contain one or more active compounds as necessary for the particular indication being treated, preferably those with complementary activities that do not adversely affect each other. In the case of anti-tumor therapeutics, such formulations may include one or a combination of addition cytotoxic agents effective for targeting the specific cell type of interest. Such molecules are suitably present in combination in amounts that are effective for the purpose intended.

The active ingredients may also be entrapped in a microcapsule prepared, for example, by coacervation techniques or by interfacial polymerization, for example, hydroxymethylcellulose or gelatin-microcapsule and poly-(methylmethacylate) microcapsule, respectively, in colloidal drug delivery systems (for example, liposomes, albumin microspheres, microemulsions, nanoparticles and nanocapsules) or in macroemulsions. Such techniques are disclosed in Remington's Pharmaceutical Sciences 16th edition, Osol, A. Ed. (1980), the contents of which are incorporated herein by reference.

In preferred embodiments, the formulations to be used for *in vivo* administration are sterile. The formulations of the instant invention can be easily sterilized, for example, by filtration through sterile filtration membranes.

Sustained-release preparations may also be prepared. Suitable examples of sustained-release preparations include semipermeable matrices of solid hydrophobic polymers containing the modified antibody, which matrices are in the form of shaped articles, e.g., films, or microcapsule. Examples of sustained-release matrices include polyesters, hydrogels (for example, poly(2-hydroxyethyl-methacrylate), or poly(vinylalcohol)), polylactides (see, e.g., U.S. Pat. No. 3,773,919, the contents of which are incorporated herein by reference), copolymers of L-glutamic acid and y ethyl-L-glutamate, non-degradable ethylene-vinyl acetate, degradable lactic acid-glycolic acid copolymers such as the LUPRON DEPOT™ (injectable microspheres composed of lactic acid-glycolic acid copolymer and leuprolide acetate), and poly-D-(-)-3-hydroxybutyric acid. While polymers such as ethylene-vinyl acetate and lactic acid-glycolic acid enable release of molecules for over 100 days, certain hydrogels release proteins for shorter time periods. When encapsulated antibodies remain in the body for a long time, they may denature or aggregate as a result of exposure to moisture at 37°C, resulting in a loss of biological activity and possible changes in immunogenicity. Rational strategies can be devised for stabilization depending on the mechanism involved. For example, if the aggregation mechanism is discovered to be intermolecular S--S bond formation through thio-disulfide interchange, stabilization may be achieved by modifying sulfhydryl residues, lyophilizing from acidic solutions, controlling moisture content, using appropriate additives, and developing specific polymer matrix compositions.

These and other aspects of the invention will be better understood by reference to the Drawings, Detailed Description, and Examples.

### EXAMPLES

### Example 1 - Materials and Methods

A. DEC-OVA: DEC-OVA and ISO-OVA in mIgG1 (D265A) form were produced as previously described in Boscardin et al., J Exp Med. 2006 Mar 20;203(3):599-606. In order to make DEC-hIgG1-OVA, DEC-hIgG1(N297A)-OVA and ISO-hIgG1-OVA, the constructs of DEC-mIgG1(D265A)-OVA and ISO-mIgG1(D265A)-OVA's heavy chains were modified so coding sequences of wild-type human IgG1 constant region or N297A mutant could replace DNA for the mouse IgG1(D265A) constant region. The constructs of DEC-mIgG1(D265A)-OVA and ISO-mIgG1(D265A)-OVA's light chains were also modified so coding sequences of DNA encoding human IgK could replace mouse IgK coding sequences. More specifically, the variable and constant regions were separately cloned in frame by PCR and ligated together by overlapping PCR using standard protocols. Full-length Ig coding sequences were digested with EcoRI/NheI and subcloned into the original DEC-mIgG1(D265A)-OVA heavy and light chain vectors.

The following primers were used for the cloning:
DEC-hIgG1-OVA and DEC-hIgG1(N297A)-OVA heavy chains:

| | |
|---|---|
| DEC_VH_F | 5'CCTCGGTTCTATCGATTGAATTCCACCATGGGATGGTCATG3' (SEQ ID NO: 11) |
| DEC_VH_R_hIgG1 | 5'CTTGGTGGAGGCTGAGGAGACTGTGACCATGACTCC3' (SEQ ID NO: 12) |
| DEC_hIgG1_F | 5'GGTCACAGTCTCCTCAGCCTCCACCAAGGGCCCATC3' (SEQ ID NO: 13) |
| DEC_hIgG1_R | 5'CTTGGCCATGTCGCTAGCTTTACCCGGAGACAGGGAGAGGC3' (SEQ ID NO: 14) |

DEC-hIgG1-OVA and DEC-hIgG1(N297A)-OVA light chains:

| | |
|---|---|
| DEC_VL_F | 5'CCTCGGTTCTATCGATTGAATTCCACCATGGGATGGTCATG3' (SEQ ID NO: 15) |
| DEC_VL_R | 5'CAGCCACAGTTCGTTTCAATTCCAGCTTGGTGCCTCC3' (SEQ ID NO: 16) |
| DEC_hIgk_F | 5'GCTGGAATTGAAACGAACTGTGGCTGCACCATCTG 3' (SEQ ID NO:17) |
| DEC_hIgk_R | 5'CAAGCTTGGGAGCGGCCGCCTAACACTCTCCCCTGTTGAAGCTCTTTG3' (SEQ ID NO: 18) |

ISO-hIgG1-OVA heavy chain:

| | |
|---|---|
| ISO_VH_F | 5'CCTCGGTTCTATCGATTGAATTCCACCATGGGATGGTCATG3' (SEQ ID NO: 19) |
| ISO_VH_R_hIgG1 | 5'CTTGGTGGAGGCTGAGGAGACTGTGACCATGACTCC3' (SEQ ID NO: 20) |
| ISO_hIgG1_F | 5'GGTCACAGTCTCCTCAGCCTCCACCAAGGGCCCATC3' (SEQ ID NO: 21) |
| ISO_hIgG1_R | 5'CTTGGCCATGTCGCTAGCTTTACCCGGAGACAGGGAGAGGC3' (SEQ ID NO: 22) |

ISO-hIgk-OVA light chain:

| | |
|---|---|
| ISO_VL_F | 5'CCTCGGTTCTATCGATTGAATTCCACCATGGGATGGTCATG3' (SEQ ID NO: 23) |
| ISO_VL_R | 5'CAGCCACAGTTCGTTTCAGTTCCAGCTTGGTCCCAGG3' (SEQ ID NO: 24) |
| ISO_hIgk_F | 5'GCTGGAACTGAAACGAACTGTGGCTGCACCATCTG 3' (SEQ ID NO: 25) |
| ISO_hIgk_R | 5'CAAGCTTGGGAGCGGCCGCCTAACACTCTCCCCTGTTGAAGCTCTTTG3' (SEQ ID NO: 26) |

DEC-OVA proteins were produced in 293T cells by transient transfection and purified by protein G Sepharose 4 Fast Flow (GE healthcare) as previously described (Boscardin et al., J Exp Med. 2006 Mar 20;203(3):599-606). LPS contamination were analyzed by Limulus Amebocyte Lysate Assay (Associates of Cape Cod, Inc.), and removed by TritonX-114 (Sigma) if necessary.
B. Anti-CD40 monoclonal antibodies (αCD40mAbs): The original anti-CD40 mAb in rat IgG2a form was secreted by hybridoma 1C10 (Heath, A.W. et al., Eur J Immunol 24, 1828 (Aug, 1994)) and purified from the culture supernatant by protein G Sepharose 4 Fast Flow (GE healthcare). In order to make αCD40mAbs of other isotypes, the heavy and light chain variable region genes were cloned by 5' RACE system according to manufacturer's instruction (Invitrogen).

The following oligonucleotides were used:

| For Heavy Chain Variable Region Gene Cloning | |
|---|---|
| HC-GSP1 | 5'ACAAGGATTGCATTCCCTTGG3' (SEQ ID NO: 27) |
| HC-GSP2 | 5'CTTGTCCACCTTGGTGCTGCT3' (SEQ ID NO: 28) |

| For Light Chain Variable Region Gene Cloning. | |
|---|---|
| LC-GSP1 | 5'CTCATTCCTGTTGAAGCTCTTGACGAC3' (SEQ ID NO: 29) |
| LC-GSP2 | 5'GGGTGAGGATGATGTCTTATGAA CA3' (SEQ ID NO: 30) |

To obtain full-length of mouse and human Ig heavy and light chain coding sequences, the heavy and light chain variable region coding sequences were cloned in frame with signal peptide by one PCR, constant region coding sequences were cloned by another PCR, and *Ig* full-length sequences were obtained by overlapping PCR using standard protocols. Full-length Ig coding sequences were then digested with EcoRI/NotI and subcloned into an expression vector driven by CMV promoter.

The following primers were used to obtain full-length Ig coding sequences:
Anti-CD40 mouse IgG1 heavy chain:

| | |
|---|---|
| 1C10_VH_F | 5'CGATTGAATTCCACCATGGACATCAGGCTCAGCTTGGTT3' (SEQ ID NO: 31) |
| 1C10_VH_R_mIgG1 | 5'GCCCTTGGTGGTGGCTGAGGAGACTGTGACCATGACTC3' (SEQ ID NO: 32) |
| 1C10_mIgG1_F | 5'GTCACAGTCTCCTCAGCCACCACCAAGGGCCCATCTGTC 3' (SEQ ID NO: 33) |
| 1C10_mIgG1_R | 5'CTTGGGAGCGGCCGCTCATTTACCAGGAGAGTGGGAGAGGCTC3' (SEQ ID NO: 34) |

Anti-CD40 mouse IgG2a heavy chain:

| | |
|---|---|
| 1C10_VH_F | 5'CGATTGAATTCCACCATGGACATCAGGCTCAGCTTGGTT3' (SEQ ID NO: 35) |
| 1C10_VH_R_mIgG2a | 5'GGCTGTTGTTTTGGCTGAGGAGACTGTGACCATGACTC3' (SEQ ID NO: 36) |
| 1C10_mIgG2a_F | 5'GTCACAGTCTCCTCAGCCAAAACAACAGCCCCATCGGTC3' (SEQ ID NO: 37) |
| 1C10_mIgG2a_R | 5'CTTGGGAGCGGCCGCTCATTTACCCAGAGACCGGGAGATGGTC3' (SEQ ID NO: 38) |

Anti-CD40 human IgG1 heavy chain:

| | |
|---|---|
| 1C10_VH_F | 5'CGATTGAATTCCACCATGGACATCAGGCTCAGCTTGGTT3' (SEQ ID NO: 39) |
| 1C10_VH_R_hIgG1 | 5'GCCCTTGGTGGAGGCTGAGGAGACTGTGACCATGACTC3' (SEQ ID NO: 40) |
| 1C10_hIgG1_F | 5'GTCACAGTCTCCTCAGCCTCCACCAAGGGCCCATCGGTC3' (SEQ ID NO: 41) |
| 1C10_hIgG1_R | 5'CTTGGGAGCGGCCGCTCATTTACCCGGAGACAGGGAGAGGCTC3' (SEQ ID NO: 42) |

Anti-CD40 mouse Igk light chain:

| | |
|---|---|
| 1C10_VL_F | 5'CGATTGAATTCCACCATGGAGACAGACAGACTCCTGCTA3' (SEQ ID NO: 43) |
| 1C10_VL_R_mIgk | 5'GCAGCATCAGCCCGTGAGGAGACTGTGACCATGACTCC3' (SEQ ID NO:44 ) |
| 1C10_mIgk_F | 5'CAAGCTGGAATTGAAACGGGCTGATGCTGCACCAACTGTA3' (SEQ ID NO: 45) |
| 1C10_mIgk_R | 5'CTTGGGAGCGGCCGCTCAACACTCATTCCTGTTGAAGCTCTTG3' (SEQ ID NO: 46) |

Anti-CD40 human Igk light chain:

| | |
|---|---|
| 1C10_VL_F | 5'CGATTGAATTCCACCATGGAGACAGACAGACTCCTGCTA3' (SEQ ID NO: 47) |
| 1C10_VL_R_hIgk | 5'GCAGCCACAGTTCGTGAGGAGACTGTGACCATGACTCC3' (SEQ ID NO: 48) |
| 1C10_hIgk_F | 5'CAAGCTGGAATTGAAACGAACTGTGGCTGCACCATCTGTC3' (SEQ ID NO: 49) |
| 1C10_hIgk_R | 5'CTTGGGAGCGGCCGCTCAACACTCTCCCCTGTTGAAGCTCTTTG3' (SEQ ID NO: 50) |

Mouse IgG1 constant region coding sequences with D265A mutation were cloned from DEC-OVA heavy chain construct by PCR using primers 1C10_mIgG1_F and 1C10_mIgG1_R. Human IgG1 constant region DNA with N297A mutation was cloned from 6A6-hIgG1(N297A) (Sazinsky et al., Proc Natl Acad Sci USA. 2008 Dec 23;105(51):20167-72) heavy chain construct using primers 1C10_hIgG1_F and 1C10_hIgG1_R. Human IgG1 constant region DNA with S267E mutation was generated by mutagenesis using the following primers:

| | |
|---|---|
| S267E_F | 5'GTGGTGGACGTGGAACACGAAGACCCT3' (SEQ ID NO: 51) |
| S267E_R | 5'AGGGTCTTCGTGTTCCACGTCCACCAC3' (SEQ ID NO: 52) |

Human IgG1 constant region DNA with G236D/S267E mutation was generated by mutagenesis on the basis of S267E mutant using the following primers:

| | |
|---|---|
| G236D_F | 5'CCTGAACTCCTGGACGGACCGTCAGTCTTCCTC3' (SEQ ID NO: 53) |
| G236D_R | 5'GAGGAAGACTGACGGTCCGTCCAGGAGTTCAGG3' (SEQ ID NO: 54) |

Human IgG1 constant region DNA with S239D/S267E mutation was generated by mutagenesis on the basis of S267E mutant using the following primers:

| | |
|---|---|
| S239D_F | 5'CTGGGGGGACCGGATGTCTTCCTCTTC3' (SEQ ID NO: 55) |
| S239D_R | 5'GAAGAGGAAGACATCCGGTCCCCCCAG3' (SEQ ID NO: 56) |

Human IgG1 constant region DNA with S267E/L328F mutation was generated by mutagenesis on the basis of S267E mutant using the following primers:

| | |
|---|---|
| L328F_F | 5'GTCTCCAACAAAGCCTTCCCAGCCCCC3' (SEQ ID NO: 57) |
| L328F_R | 5'GGGGGCTGGGAAGGCTTTGTTGGAGAC3' (SEQ ID NO: 58) |

Human IgG1 constant region DNA with S239D/I332E mutation was generated by mutagenesis using the following primers:

| | |
|---|---|
| S239D_F | 5'CTGGGGGGACCGGATGTCTTCCTCTTC3' (SEQ ID NO: 59) |
| S239D_R | 5'GAAGAGGAAGACATCCGGTCCCCCCAG3' (SEQ ID NO: 60) |
| I332E_F | 5'GCCCTCCCAGCCCCCGAAGAGAAAACCATCTCC3' (SEQ ID NO: 61) |
| I332E_R | 5'GGAGATGGTTTTCTCTTCGGGGGCTGGGAGGGC3' (SEQ ID NO: 62) |

Anti-CD40 mAbs were produced in 293T cells by transient transfection and purified by protein G Sepharose 4 Fast Flow (GE healthcare). LPS contamination were analyzed by Limulus Amebocyte Lysate Assay (Associates of Cape Cod, Inc.), and removed by TritonX-114 (Sigma) if necessary.

F(ab')2 fragment of αCD40mAb was made using F(ab')2 preparation kit (Pierce) following manufacturer's instruction. Intact and F(ab')2 fragments were examined on SDS-PAGE gel (NuPAGE, 4-12% Bis-Tris Mini Gels, Invitrogen) in non-reducing conditions.

In order to prepare aglycosylated αCD40mAb, intact αCD40mAb in rat IgG2a form were treated with EndoS (IgGZERO, Genovis) following manufactuer's instruction, and purified by protein G Sepharose 4 Fast Flow (GE healthcare). The efficiency of EndoS treatment was examined by *Lens culinaris* agglutinin (LCA, Vector Laboratory) lectin blot as previously described.
C. Mice: Wild-type B6 mice were purchased from Taconic. The FcγRIIb-deficient (R2^{-/-}) mice and the FcR common γ-chain deficient mice (γ-/-) were generated in our laboratory. They were either generated on pure B6 genetic background or had been backcrossed to C57BL/6 background for more than 12 generation. FcγR-deficient mice ((γR2)^{-/-}) were generated by breeding R2^{-/-} and γ^{-/-} mice. Human FcγRIIa and FcγRIIb BAC transgenic mice were generated in our laboratory on B6 genetic background, and bred with R2^{-/-} and (γR2)^{-/-} mice to generated R2^{-/-}hRIIb⁺ and (γR2)^{-/-}hRIIa⁺hRIIb⁺ mice, respectively. Two-three month old sex-matched mice were used in all experiments. Mice are maintained in The Rockefeller University Animal Facility. All experiments were performed in compliance with federal laws and institutional guidelines and had been approved by the Rockefeller University IACUC
D. OVA-specific T cell response: Two to three months sex matched mice were immunized with 5µg of DEC-OVA or ISO-OVA in different forms (human IgG1 and its N297A mutant, and mouse IgG1 D265A mutant) with or without 30µg (or other stated amount) of αCD40mAb in different forms (untreated rat IgG2a antibody, EndoS-treated rat IgG2a, or 20µg of F(ab')2 fragment of rat IgG2a antibody; mouse IgG1, IgG2a, or IgG1 D265A mutant; human IgG1, IgG1 N297A, IgG1 S267E, IgG1 S239D/I332E, IgG1 G236D/S267E, IgG1 S239D/S267E, or IgG1 S267E/L328F mutants). Seven days later, spleens were isolated or peripheral blood was collected. Single cell suspension was prepared following lysing red blood cells. Two analyses were exploited to quantify the expansion of OVA peptide SIINFEKL-specific T cells:

Tetramer staining: spleen cells resuspended in FACS buffer (PBS with 0.5% FBS, 2mM EDTA and 0.1%NaN3) were stained with fluorescent-labeled anti-CD4 and anti-CD8a antibodies (BD Biosciences), and OVA peptide SIINFEKL tetramer (Tet-OVA, H-2^{b} with OVA peptide SIINFEKL, Beckman Coulter) for 30minutes on ice then 30 minutes at room temperature. After two washes with FACS buffer, 7AAD was added before analysis to exclude dead cells.

*In vitro* stimulation and intracelluar IFN-γ staining: spleen cells were cultured in media (RPMI with 10% FBS, 1% Pen Strep, 10mM HEPES, 50µM 2-Mercaptoethanol) with 1µg/ml anti-CD28 antibody and 1µg/ml OVA peptide SIINFEKL for 6 hours. Brefeldin A was added 1 hour after the culture started to a final concentration of 10µg/ml. Cultured spleen cells were stained for surface CD4 and CD8a for 15 minutes on ice, followed by intracellular IFN-γ staining using manufacturer's protocol (BD biosciences).

FACS data were acquired on FACScan (BD biosciences) and analyzed by Flowjo.
E. MO4 tumor model: MO4, an OVA-expressing melanoma cell line (Bonifaz et al., J Exp Med. 2004 Mar 15;199(6):815-24) was cultured in DMEM with 10% FBS, 1% Pen Strep, and 0.4mg/ml neomycin. (γR2)^{-/-}hRIIb⁺ mice were inoculated with 10⁷ MO4 cells in 50µl PBS subcutaneously on the flank. Ten days later (when diameters of tumors were about 5∼10mm), mice were treated with 5µg of DEC-OVA (in human IgG1 N297A mutant form) and 30µg of αCD40mAb in different forms (human IgG1, human IgG1 N297A and S267E mutants). Tumor growth was monitored every other day after treatment. Area was calculated as *π*d²/4 where "d" is diameter.
F. A20 tumor model: A20 cells were maintained in RPMI with 10% FBS, 1% Pen Strep, 1mM Sodium Pyruvate, 10mM HEPES, and 50µM 2-Mercaptoethanol (INVITROGEN). BALB/c mice were injected intravenously with either 200µg of mouse control IgG, or CD40 antibodies with different mouse IgG Fc's (mouse IgG1 Fc or its D265A variant, or mouse IgG2a Fc). One hour later, 2x10⁷ A20 cells were inoculated subcutaneously. Tumor growth was monitored and tumor area values were calculated as *π*d²/4 where "d" was the diameter of the tumors.
G. B6BL and B6BL-CD40 models: B6BL is a spontaneous B cell lymphoma isolated from p53^{fl/fl}CD19Cre⁺ mice on pure B6 genetic background (Li and Ravetch, Science. 2011 Aug 19;333(6045):1030-4). B6BL cells were maintained in RPMI with 10% FBS, 1% Pen Strep, 1mM Sodium Pyruvate, 10mM HEPES, and 50µM 2-Mercaptoethanol (INVITROGEN). FACS analysis of B6BL surface phenotype showed that B6BL cells were CD19⁺FcγRIIb⁺IgM⁻ CD40⁻, therefore represented a pro/pre-B cell lymphoma line. In order to generate CD40-expressing B6BL (B6BL-CD40), CD40 isoform1 (Genbank Acc#: NM_011611) cDNA was cloned from wild-type C57BL/6 spleen RNA by SuperScript® III one-Step RT-PCR System with Platinum® Taq High Fidelity (INVITROGEN), into pFB-neo retroviral vector (STRATAGENE). Retroviral particles were produced in 293T cells and used to transduce B6BL cells. Transduced B6BL cells were selected by 1mg/ml Geneticin for 2 weeks and sorted for CD40⁺ cells. Sorted CD40⁺ cells, referred to as "B6BL-CD40" in this study, were maintained in RPMI with 10% FBS, 1% Pen Strep, 1mM Sodium Pyruvate, 10mM HEPES, 50µM 2-Mercaptoethanol, and 0.4mg/ml Geneticin (INVITROGEN). The following primers were used to clone CD40 isoform1 cDNA:

| | |
|---|---|
| mCD40_F | 5'AATTGTCGACCACCATGGTGTCTTTGCCTCGGCTGTGC3' (SEQ ID NO: 63) |
| mCD40_R | 5'AATTGCGGCCGCTCAGACCAGGGGCCTCAAGGCTATG3' (SEQ ID NO: 64) |

In B6BL and B6BL-CD40 models, B6, FcγRIIb-deficient (R2^{-/-}), human FcγRIIb-transgenic R2^{-/-} mice (R2^{-/-}hRIIb⁺), FcR common-y chain deficient mice (γ^{-/-}) were inoculated with 2.5X10⁷ tumor cells intravenously on day 0, and treated with a first dose of control IgG or CD40 antibodies with one of the various mouse or human IgG Fc's (mouse IgG1 Fc or its D265A variant, mouse IgG2a Fc, human IgG1 Fc or its N297A or S267E variants) on day 3 and the second dose on day 4 or 6 by *i.v.* injection. Each treatment used 200µg of mouse or human control IgG, or CD40 antibodies except where lower dosage treatments (40µg of αCD40:hIgG1 or αCD40:hIgG1(S267E) per dose) were studied. In some experiments, 200µg (per dose) of αCD8 depleting antibodies (clone 2.43, Bio X Cell) were also included in the treatment. Mice were monitored daily for survival. In some experiments, long-time survivors were re-challenged with 2.5X10⁷ B6BL cells 10 weeks after the initial B6BL inoculation.
H. CD40 antibodies induced ADCC and CD8⁺ T cell expansion: WT mice were treated with 200µg of control mouse IgG, or CD40 antibodies with different mouse IgG Fc's (moue IgG1 Fc or its D265A variant, or mouse IgG2a Fc). Six days later, peripheral blood samples were collected and treated with red blood cell lysing buffer (BD biosciences). The percentage of CD40⁺ cells and ratio of CD8⁺ to CD4⁺ T cells were analyzed by FACS using fluorescent-labeled anti-CD4, anti-CD8α, and anti-CD40 (1C10). 7AAD was added to exclude dead cells.
I. Anti-DR5 monoclonal antibodies: The hamster anti-mouse DR5 antibodies, clone MD5-1 (Takeda et al., J Exp Med. 2004 Feb 16;199(4):437-48), were either purchased from Bio X Cell or purified from the culture supernatant of MD5-1 hybridoma cells by protein G Sepharose 4 Fast Flow (GE healthcare). In order to make DR5 antibodies with different Fc's, the heavy and light chain variable region genes were cloned by 5' RACE system according to manufacturer's instruction (Invitrogen).

The following oligonucleotides were used:

| For Heavy Chain Variable Region Gene Cloning | |
|---|---|
| HC-GSP1 | 5'GCTCACGTCCACCACCACACATGT3' (SEQ ID NO: 65) |
| HC-GSP2 | 5'GAAATAGCCCTTGACCAGGCATCC3' (SEQ ID NO: 66) |

| For Light Chain Variable Region Gene Cloning. | |
|---|---|
| LC-GSP1 | 5'CTAACACTCATTCCTGTTCAGGGTCTTG3' (SEQ ID NO: 67) |
| LC-GSP2 | 5'GCTGCTCAGGCTGTAGGTGCTGTC3' (SEQ ID NO: 68) |

Full-length of Ig heavy and light chain coding sequences were cloned using the same method described in the CD40 antibody section, with the following primers: Anti-DR5 human IgG1 heavy chain:

| | |
|---|---|
| MD5-1_VH_F | 5'CGATTGAATTCCACCATGAGACTGCTGGGTCTTCTGTACCTG3' (SEQ ID NO: 69) |
| MD5-1_VH_R_hIgG1 | 5'GCCCTTGGTGGAGGCTGAGGAGACGGTGACCAGGGTCCC3' (SEQ ID NO: 70) |
| MD5-1_hIgG1_F | 5'GTCACCGTCTCCTCAGCCTCCACCAAGGGCCCATCGGTC3' (SEQ ID NO: 71) |
| MD5-1_hIgG1_R | 5'CTTGGGAGCGGCCGCTCATTTACCCGGAGACAGGGAGAGGCTC3' (SEQ ID NO: 72) |

Anti-CD40 human Igk light chain:

| | |
|---|---|
| MD5-1_VL_F | 5'CGATTGAATTCCACCATGGCCATGAAGGTTCCTGCTCA3' (SEQ ID NO: 73) |
| MD5-1_VL_R_hIgk | 5'GCAGCCACAGTTCGTTTGATTTCCAGTCTGGTCCCTCC3' (SEQ ID NO: 74) |
| MD5-1_hIgk_F | 5'CAGACTGGAAATCAAACGAACTGTGGCTGCACCATCTGTC3' (SEQ ID NO: 75) |
| MD5-1_hIgk_R | 5'CTTGGGAGCGGCCGCTCAACACTCTCCCCTGTTGAAGCTCTTTG3' (SEQ ID NO: 76) |

Human IgG1 constant region DNA with N297A or S267E mutations was cloned from anti-CD40 antibody constructs by PCR using primers MD5-1_hIgG1_F and MD5-1_hIgG1_R.

DR5 antibodies with human IgG1 Fc were produced in 293T cells by transient transfection and purified by protein G Sepharose 4 Fast Flow (GE healthcare). LPS contamination were analyzed by Limulus Amebocyte Lysate Assay (Associates of Cape Cod, Inc.), and removed by TritonX-114 (Sigma) if necessary.
J. Liver toxicity studies. Two treatment protocols were used to study liver toxicity induced by DR5 antibodies. In one protocol, wild-type C57BL/6 and Fcγ-deficient (R2^{-/-} ) mice were treated 300µg of hamster IgG (hamIgG) or MD5-1 through intravenous injection on days 0, 3, 6, and 9. Serum samples were collected on day 14 and analyzed for aspartate transaminase (AST) and alanine transaminase (ALT) levels. Mice were monitored for two months for survival. In the other protocol, human FcγRIIb-transgenic mice on FcγR-deficient background ((γR2)^{-/-} hRIIb⁺) were treated with a single dose of 100µg of human IgG, or DR5 antibodies with unmutated human IgG1 Fc, or its N297A or S267E variants, and analyzed for AST and ALT levels 7 days later. AST and ALT enzymatic kits (BIOO Scientific Corp., Austin, TX) were used to determine AST and ALT levels.
K. MC38 tumor models. Wild-type C57BL/6 and FcγRIIb-deficient (R2^{-/-}) were inoculated with 10⁶ MC38 cells subcutaneously. After MC38 tumors were established, groups of 5 mice were treated with 100µg of hamster IgG or MD5-1 antibodies through intravenous injection, on day 7, 11, and 15. Tumor growth was monitored every four days.

### Example 2 - Anti-CD40 mAb requires FcγRs for its adjuvant activity

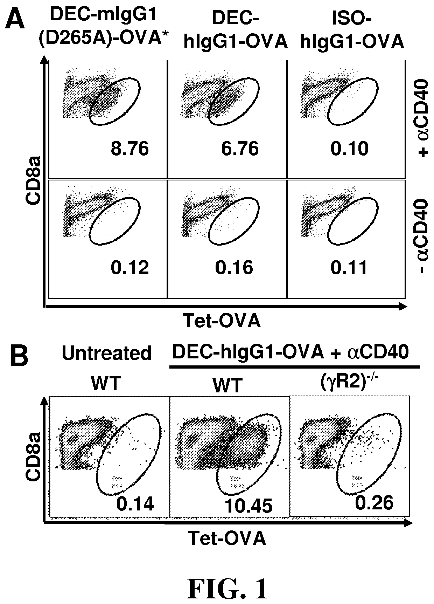

In order to investigate whether FcyRs regulate adjuvant effect of an agonistic anti-CD40 monoclonal antibody (αCD40mAb, clone 1C10, rat IgG2a), the targeted antigen delivery system established at The Rockefeller University was exploited. This system uses anti-DEC-205 antibody to deliver model antigen OVA fused to the C-terminal of the antibody, and uses this agonistic αCD40mAb as adjuvant. FIG. 1 illustrates that FcγRs are required for OVA-specific T cell response induced by DEC-hIgG1-OVA (OVA fused to an anti-DEC205 antibody with human IgG1 constant region) and αCD40mAb. As previously reported and confirmed in FIG. 1A, mice immunized with DEC-OVA (OVA fused to anti-DEC205 antibody) develop OVA-specific CD8⁺ T cell response that was detected by both OVA-peptide SIINFEKL tetramer (Tet-OVA, H-2^{b} with OVA peptide SIINFEKL) staining and intracellular IFN-γ staining following *in vitro* T cell stimulation. But this response is only observed in the presence of adjuvant such as αCD40mAb.

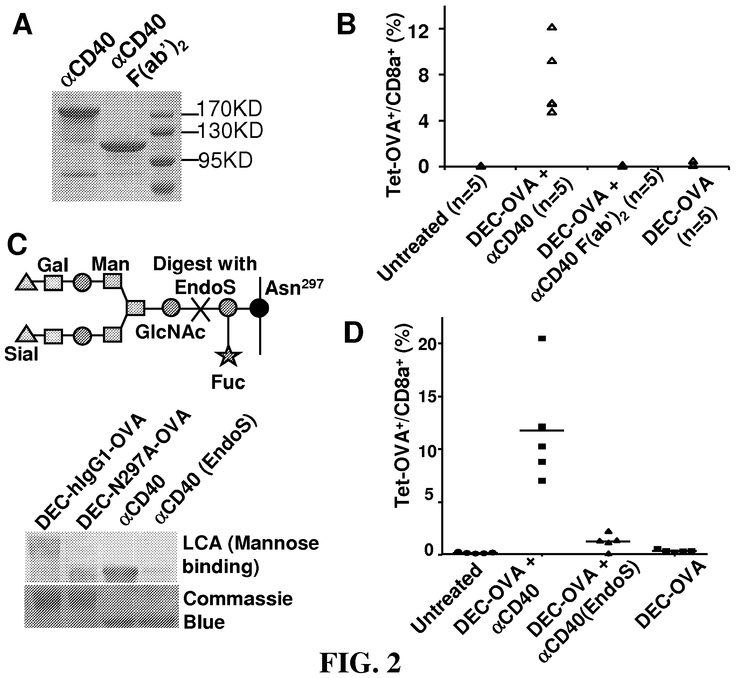

Whether adjuvant effect of αCD40mAb requires FcγRs were tested by comparing wild-type B6 mice and FcγR-deficient mice (γ-chain and FcγRIIb double knockout, deficient for all FcyRs) with B6 genetic background in DEC-OVA system. As shown in FIG. 1B, FcγR-deficient mice failed to develop OVA-specific CD8a⁺ T cell response, suggesting that FcγRs were required in this response. Although both DEC-hIgG1-OVA and αCD40mAb, the two antibodies used in this experiment, have the potential to interact with mouse FcγRs, DEC-hIgG1-OVA is unlikely the one that requires FcγR-interaction because the model was established using DEC-OVA of mouse IgG1 isotype with D265A mutation that abrogates all FcγR-binding. The efficiency of DEC-OVA in mouse IgG1 D265A form to induce OVA-specific T cell response was confirmed in wild-type B6 mice (FIG. 1A). DEC-hIgG1(N297A)-OVA, another DEC-OVA mutant form that doesn't binds FcγRs due to a lack of N297 glycosylation site in human IgG1, can also replace DEC-hIgG1-OVA in the DEC-OVA response. See Li and Ravetch, Science. 2011 Aug 19;333(6045):1030-4, the content of which is incorporated by reference. These data suggest that it is αCD40mAb that requires FcγR-interaction. In order to test this hypothesis, we prepared F(ab')2 fragment and deglycosylated form of αCD40mAb that can not bind FcγRs (Allhorn, M. et al., PLoS One 3, e1413 (2008)). (FIG. 2A, 2C).

Shown in FIG. 2A, is a non-reducing SDS-PAGE gel prepared from αCD40 F(ab')2 Preparation Kits. As illustrated in FIG. 2B, the Fc portion of αCD40mAb is required for its adjuvant effect. Wild-type B6 mice were immunized with 5µg of DEC-OVA (DEC-hIgG1(N297A)-OVA) plus intact 30µg of αCD40mAb or 20µg of αCD40 F(ab')2 fragment, and analyzed as described herein. The percentage of OVA-specific CD8a⁺ T cells in spleen (determined by OVA peptide tetramer (H-2^{b} with SIINFEKL OVA peptide) staining) was plotted. Each triangle represents a mouse. FIG. 2C illustrates the preparation of deglycosylated αCD40mAb. Illustrated in the upper panel is the glycosylation structure at N297 site and the EndoS cleavage site between the 2nd and 3rd GlcNAc. Illustrated in the lower panel is the LCA lectin blot (specific for mannose) and commassie blue staining of DEC-hIgG1-OVA and its aglycosylation mutant (DEC-N297A-OVA), αCD40mAb and endoS-treated αCD40mAb. FIG. 2D illustrates that an EndoS-treated αCD40mAb has no adjuvant effect. Wild-type B6 mice were immunized and analyzed as stated herein, except endoS treated αCD40mAb was tested. In FIGs. 2B and 2D, neither F(ab')2 nor deglycosylated form of αCD40mAb supported DEC-OVA response, suggesting no adjuvant effect for αCD40mAb without FcyR-binding capacity. These data demonstrated that αCD40mAb requires FcγR-interaction for its adjuvant effect.

### Example 3 - FcγRIIb provides necessary FcγR-interaction for αCD40mAb's adjuvant effect

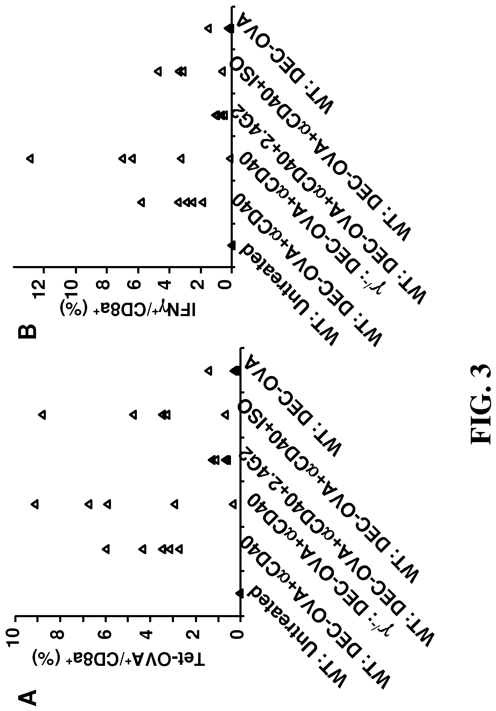

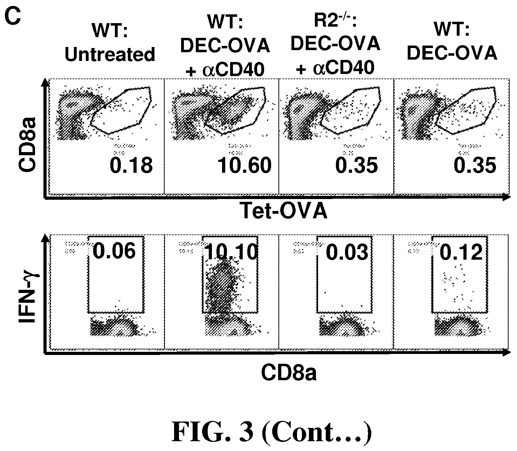

In order to further characterize the FcγR-requirement for αCD40mAb's adjuvant effect, mice with mutations in FcγR genes were tested in the DEC-OVA model. As shown in FIGs. 3A and 3B, the FcR common γ-chain deficient mice (γ^{-/-}) had no defect in the OVA-specific T cell response induced by DEC-OVA and αCD40mAb, suggesting all activating FcyRs were dispensable. In contrast, FcγRIIb knockout mice were completely defective in this response (FIG. 3C), and this defect was not due to any developmental problems in these mice as 2.4G2 blocking antibody for FcγRIIb and FcγRIII can block this response in wild-type B6 mice (FIGs. 3A and 3B). Taken together, these data demonstrated that αCD40mAb's adjuvant effect requires FcγRIIb-interaction.

FIG. 3A provides a graph showing the percentage of Tet-OVA⁺ in splenic 7AAD⁻ CD8a⁺CD4⁻ cells. Wild-type B6 and (γ^{-/-}) mice were immunized with 5 µg of DEC-OVA and 30 µg of αCD40mAb, 5 µg of DEC-OVA and 30 µg of αCD40mAb plus 100 µg of 2.4G2 blocking antibody or isotype control, or 5 µg of DEC-OVA alone. OVA peptide SIINFEKL-specific T cells in spleen were analyzed by tetramer staining (FIG. 3A) and intracellular IFN-γ staining after *in vitro* stimulation (FIG. 3B). Each triangle represents % of a mouse. In the upper panel of FIG. 3C, FACS profiles are illustrated showing the percentage of splenic Tet-OVA⁺ cells (gated on 7AAD⁻CD8a⁺CD4⁻ cells). In the lower panel, FACS profiles showing the percentage of splenic IFN-γ⁺ cells (gated on CD8a⁺CD4⁻ cells) are illustrated. Wild-type B6 mice and FcγRIIb-deficient mice (R2^{-/-}) were immunized with 5 µg of DEC-OVA and 30 µg of αCD40mAb, or 5 µg of DEC-OVA alone. The percentage of OVA peptide SIINFEKL-specific T cells in spleen was analyzed by both tetramer and intracellular IFN-γ staining.

### Example 4 - Modulate αCD40mAb's adjuvant effect by manipulating its FcγR-bindng

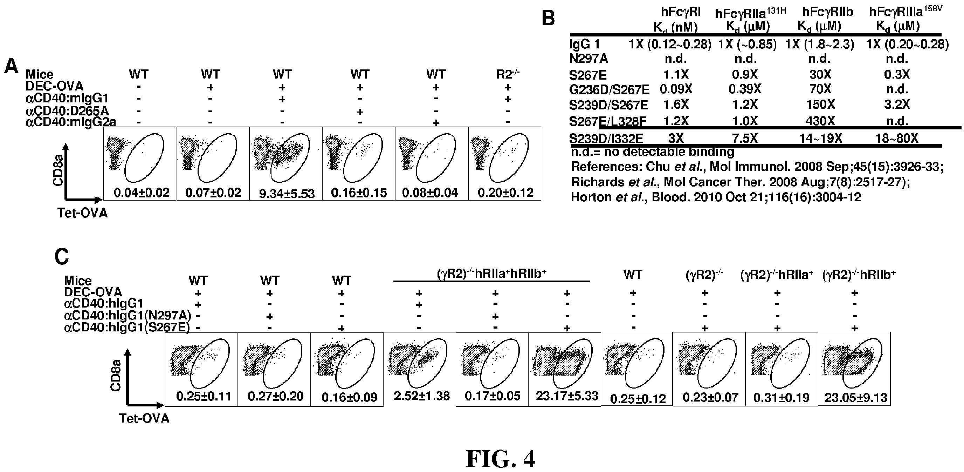

The possibility to modulate adjuvant effect of αCD40mAb by manipulating its FcγRIIb-binding was also tested in DEC-OVA model. Because mouse IgG1 and IgG2a have the highest binding affinity to mouse FcγRIIb and activating FcγRs, respectively, αCD40mAb in mouse IgG1 and mouse IgG2a forms were produced and purified, as well as αCD40mAb in mouse IgG1 D265A mutant form that does not bind FcγRs. These αCD40mAbs were compared in DEC-OVA model in wild-type B6 mice. As shown in FIG. 4A, αCD40mAb in mouse IgG2a form with low FcγRIIb-binding affinity had no detectable adjuvant effect. In contract, αCD40mAb in mouse IgG1 form with about 10 fold higher affinity to FcγRIIb (as compared to mouse IgG2a) showed strong adjuvant effect. The adjuvant effect of αCD40mAb in mouse IgG1 form also requires FcγRIIb-interaction as either D265A mutation or the lack of FcγRIIb abrogated its adjuvant effect. These data demonstrated that the affinity of αCD40mAbs to FcγRIIb affects their adjuvant effect.

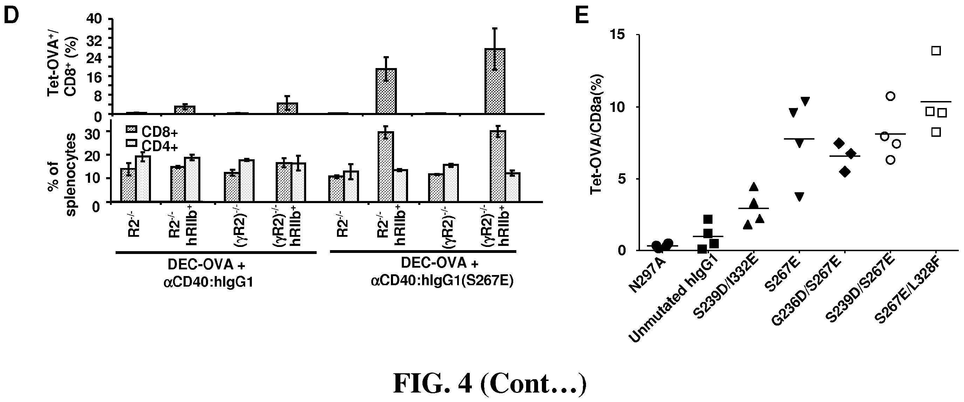

In order to further test this hypothesis, αCD40mAbs in human IgG1 form and variants with either enhanced or abrogated binding to human FcγRIIb were produced and tested in mice with human FcγRIIa and FcγRIIb transgenes (FIGs. 4B, C, and D). As shown in FIG. 4C, human FcγRIIa transgene did not support αCD40mAb in human IgG1 form. Similar assays were carried out using mouse FcγRs transgene and it was found that mouse FcγRs transgene did not support αCD40mAb in human IgG1 form or its variants, which might be explained by low affinity between IgG and FcγRs of different species (*i.e.* mouse versus human). Consistently, human FcγRIIb expressed in transgenic mice supported αCD40mAb in human IgG1 form, but not the human IgG1 N297A mutant form of αCD40mAb without FcγRIIb-binding capacity (FIG. 4C). Furthermore, αCD40mAb with enhanced human FcγRIIb binding (human IgG1 S267E mutant) had strongly increased adjuvant effect (FIGs. 4C and D) and induced a large expansion of splenic CD8⁺ T cells (FIG. 4D). αCD40mAbs with several other FcyRIIb-enhanced human IgG1 Fc variants (Chu et al., Mol Immunol. 2008 Sep;45(15):3926-33 and Richards et al., Mol Cancer Ther. 2008 Aug;7(8):2517-27) were also tested for their adjuvant potency in (γR2)^{-/-}hRIIb⁺ mice. As shown in FIG. 4E, αCD40mAbs with human IgG1 Fc containing one of the following mutation combinations, S239D/I332E, G236D/S267E, S239D/S267E, S267E/L328F, all showed increased adjuvant activities as compared the unmutated version. These studies demonstrated that adjuvant effect of αCD40mAb can be modulated by manipulating FcγRIIb-interaction.

FIG. 4A illustrates that wild-type B6 mice and FcγRIIb-deficient mice (R2^{-/-}) were immunized with 5 µg of DEC-OVA (in human IgG1 N297A mutant form) and 30 µg of αCD40mAb (in forms of rat IgG2a, mouse IgG1, mouse IgG2a, and mouse IgG1 D265A mutant), or 5 µg of DEC-OVA alone. The percentage of OVA peptide SINFEKL-specific T cells in peripheral blood was analyzed 7 days later by tetramer staining. FIG. 4B provides a table showing the affinity of human IgG1 and its variants to human FcγRI, FcγRIIb, FcγRIIa, and FcγRIIIa. FIG. 4C illustrates that wild-type B6, FcyR-deficient ((γR2)^{-/-}, (γR2)^{-/-}hRIIa⁺, (γR2)^{-/-}hRIIb⁺, and (γR2)^{-/-}hRIIa⁺, (γR2)^{-/-}hRIIb⁺ mice were immunized with 5 µg of DEC-OVA (in human IgG1 N297A mutant form) and 30 µg of αCD40mab (in forms of wild-type human IgG1, human IgG1 N297A and S267E mutants), or 5 µg of DEC-OVA alone. In the upper panel of FIG. 4D, a bar graph is illustrated showing the percentage of Tet-OVA⁺ in splenic 7AAD⁻ CD8a⁺CD4⁻ cells. In the lower panel, a bar graph is illustrated showing the percentage of CD4⁺ and CD8a⁺ splenic cells. FcγRIIb-deficient (R2^{-/-}), (R2^{-/-}hRIIb⁺), FcyR-deficient ((γR2)^{-/-}), (γR2^{-/-}hRIIb⁺) mice were immunized with 5 µg of DEC-OVA (in human IgG1 N297A mutant form) and 30 µg of αCD40mAb (in forms of wild-type human IgG1 and human IgG1 S267E mutant). The percentage of OVA peptide SIINFEKL-specific T cells, CD4⁺ and CD8⁺ T cells in the spleen was analyzed by FACS with tetramer (Tet-OVA), anti-CD4 and anti-CD8 antibodies. FIG. 4E shows that a panel of αCD40mAbs with increased human FcγRIIb-binding affinities has enhanced adjuvant activities. More specifically, (γR2)^{-/-}hRIIb⁺ mice were immunized with 5µg of DEC-OVA and 10µg of human IgG1 αCD40mAbs with the indicated mutations. The percentage of OVA peptide SIINFEKL-specific CD8⁺ T cells was analyzed in peripheral blood 7 days after immunization and presented.

### Example 5 - Antitumor activity of DEC-OVA and αCD40mAb can be modulated by manipulating FcγR-binding

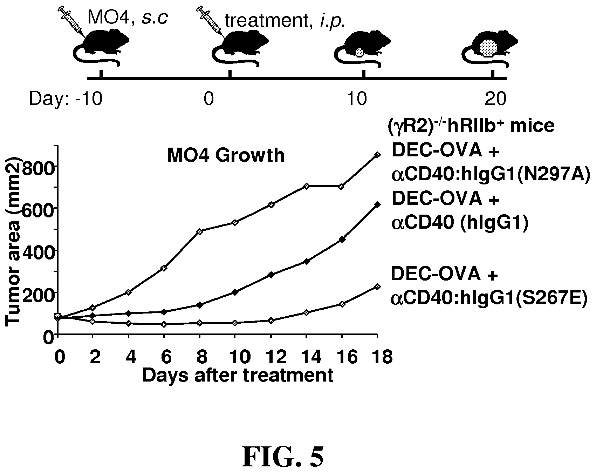

In order to test whether αCD40mAb with enhanced FcγRIIb binding has enhanced antitumor effect, mice with human FcγRIIb transgene and without endogenous FcyRs ((γR2)^{-/-}hRIIb⁺) were used in a melanoma tumor model. OVA-expressing melanoma tumor cells (MO4) were inoculated and 10 days later when tumor diameters were about 10mm, mice were treated with DEC-OVA and αCD40mAb with different human FcγRIIb binding affinity (wild-type human IgG1, S267E, or N297A variants). As shown FIG. 5, αCD40mAbs in both wild-type and enhanced human IgG1 forms have protective effect (as compared to αCD40mAb without FcγR-binding), but αCD40mAb with enhanced FcγRIIb has much higher antitumor activity.

FIG. 5 illustrates that αCD40mAb with enhanced human FcγRIIb binding has increase antitumor activity. In the upper panel, diagram is provided showing experiment setup for therapeutic MO4 tumor model. Red area represents tumor. In the lower panel, a chart is provided showing tumor growth curve (as measured by area) in mice received indicated treatments. Representative of three mice. MO4 tumor cells were inoculated in (γR2)^{-/-} hRIIb⁺mice by s.c. injection, 1x10⁷ cells per mouse. Ten days later, when the tumor area was about 100mm², mice were treated with 5µg of DEC-OVA (in human IgG1 N297A mutant form) and 30µg of αCD40mAb in forms of wild-type human IgG1, human IgG1 N297A mutant, or human IgG1 S267E mutant. Tumor growth was monitored every other day after treatment. Area was calculated as *π*d²/4 where d=diameter.

### Example 6 - Anti-tumor effect of αCD40 in a CD40 negative B6 B cell lymphoma (B6B1) model

A spontaneous, CD40 negative, B cell lymphoma cell line derived from p53LoxP, CD19Cre⁺ mice on the B6 background was provided by M. Nussenzweig (Rockefeller University). B6BL cells were maintained in RPMI media with 10% FBS, 2mM L-glutamine, 10mM HEPES, 1mM Sodium Pyruvate, 50µM 2-Mercaptoethanol, and antibiotics. To inoculate tumors, 2 or 2.5x10⁷ B6BL cells per mouse were injected through the tail vein on day 0. These mice (wild-type B6, R2^{-/-}, and R2^{-/-}hRIIb⁺) were treated on day 3 and day 4 with PBS, mouse or human IgG, or αCD40mAbs of different forms (mouse IgG1, mouse IgG1 with D265A mutation, mouse IgG2a, human IgG1, human IgG1 with N297A mutation, and human IgG1 with S267E mutation), and monitored for survival.

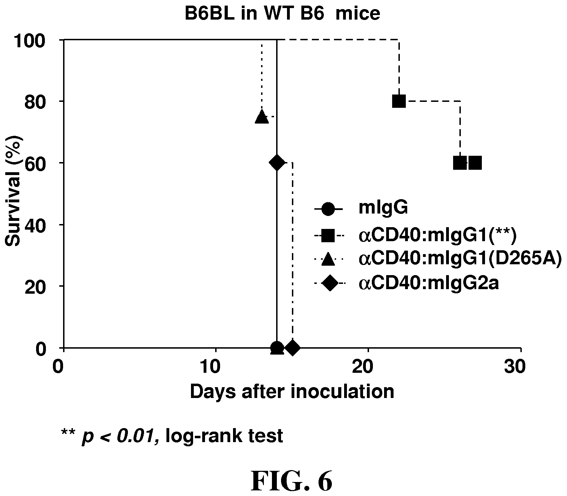

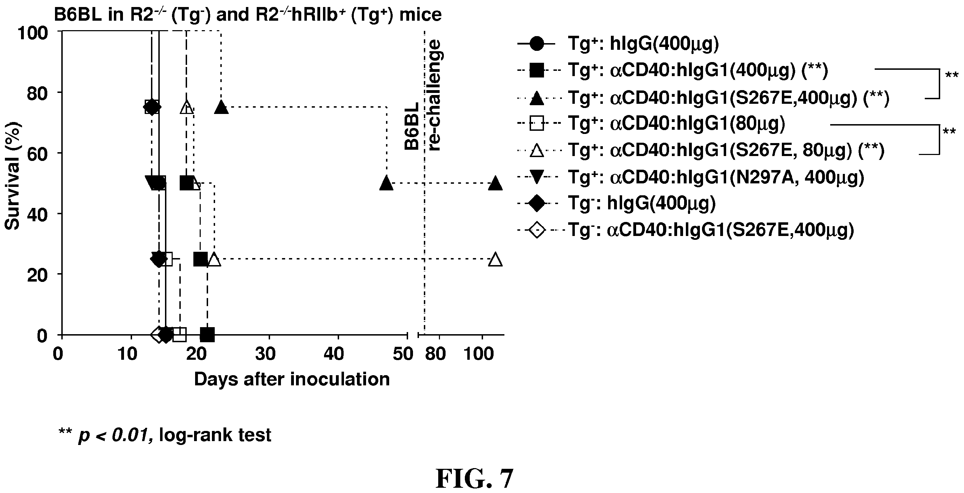

Anti-tumor effect of αCD40mAb was also studied in the CD40 negative B6 B cell lymphoma. Mouse IgG versions of αCD40mAbs (mIgG1, mIgG1- D265A mutant, and IgG2a) were compared in wild-type B6 mice using this model, as shown in FIG. 6, anti-tumor effects were only detected with the mouse IgG1 form αCD40mAb, not the D265A mutant or IgG2a forms, correlating with their adjuvant activities. Humanized αCD40mAbs were tested in FcyRIIb-humanized mice (R2^{-/-}hRIIb⁺, FcγRIIb-deficient mice with human FcγRIIb transgene). As shown in FIG. 7, αCD40mAb in human IgG1 form had weak anti-tumor effects, and the N297A mutant form of αCD40mAb with FcγR binding-deficiency had no detectable anti-tumor effect. In contrast, αCD40mAb with enhanced human FcγRIIb-binding (S267E) was at least 5 fold more protective than the original human IgG1 form. Comparing the anti-tumor effect of the S267E mutant αCD40mAb in R2^{-/-} and R2^{-/-}hRIIb⁺ mice showed that its antitumor effect was human FcγRIIb-transgene dependent. Furthermore, it was found that re-challenge of the B6BL-surviving animals at 10 weeks with B6BL tumor cells resulted in resistance, indicating the presence of a memory response. These results demonstrated that in the absence of the target tumor antigens, αCD40mAb displayed a strong adjuvant effect, which, by itself, is powerful enough to boost the host system to target tumor cells and inhibit tumor growth. These results also consistently showed that anti-tumor effect of αCD40mAb correlates with its adjuvant effect.

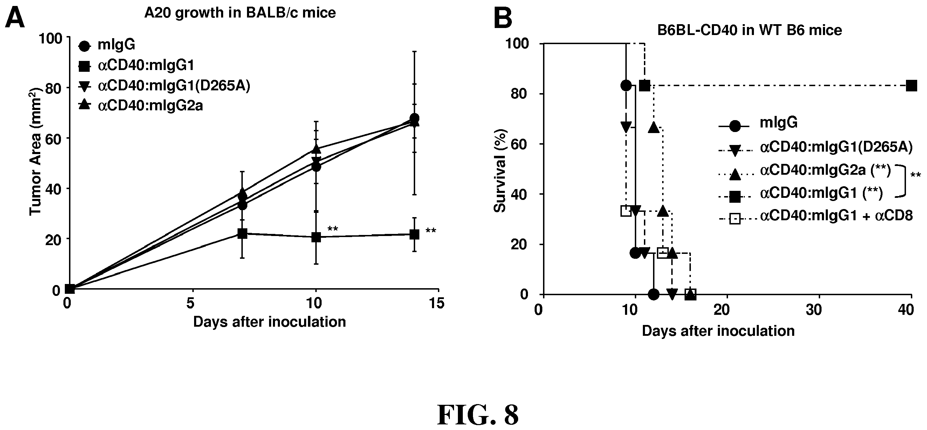

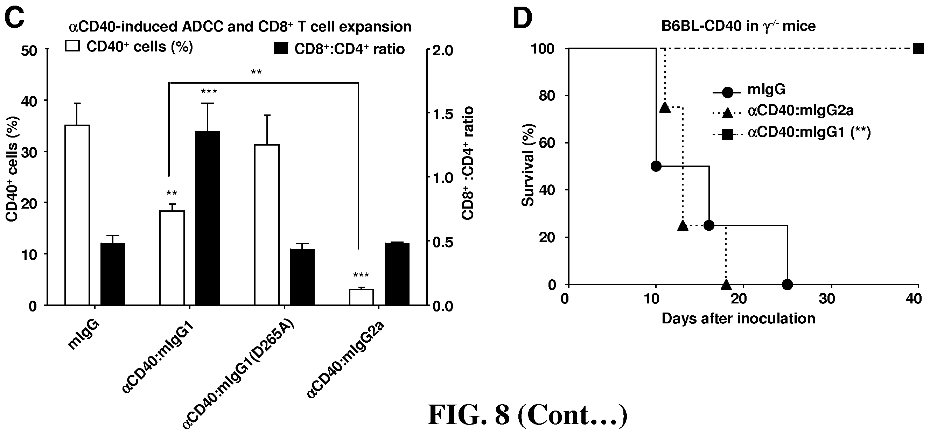

Anti-tumor efficacy of αCD40mAbs that target activating or inhibitory Fcγ receptors was further compared in two different CD40⁺ tumor models, the BALB/c-derived A20 lymphoma and B6BL-CD40 (B6BL engineered to express CD40). Wild-type C57BL/6 or BALB/c mice were challenged with A20 or B6BL-CD40 tumors, respectively, and treated with agonistic αCD40mAbs enhanced for either ADCC (αCD40:mIgG2a) or FcγRIIb-binding (αCD40:mIgG1) (FIGs. 8A and B). αCD40:mIgG2a treatment showed no effect on A20 growth and a small, but significant improvement in survival in B6BL-CD40 challenged mice at the dose indicated, while treatment at the same dose of αCD40:mIgG1 resulted in arrest of tumor growth for A20 (FIG. 8A) or long-term survival for B6BL-CD40 challenged mice (FIG. 8B). The lack of anti-tumor activity for αCD40:mIgG2a was not due to defects in ADCC activity since the antibody displayed robust depletion of peripheral CD40⁺ cells in treated mice (FIG. 8C). In contrast, αCD40:mIgG1 treated mice displayed marked expansion of CD8⁺ cells in the periphery (FIG. 8C). The anti-tumor effect of αCD40:mIgG1 in prolonging survival of B6BL-CD40 challenged mice was not affected by deficiency in FcRγ chain (required for all activating FcyRs, FIG. 8D), supporting an ADCC-independent mechanism for this anti-tumor effect. In addition, depleting CD8⁺ cells abrogated the anti-tumor effect of αCD40:mIgG1, confirming a CD8⁺ T cell-mediated anti-tumor mechanism (Fig. 8B). These data demonstrated that the adjuvant effects of agonistic CD40 mAbs (activation of cytotoxic T cells through CD40 mediated stimulation of APCs) results in a more potent anti-tumor effect than cytotoxicity triggered through effector cell activation through FcγR crosslinking.

Specifically, as shown in FIG. 8A, wild-type BALB/c mice were treated with the indicated CD40 antibodies or control IgG (200 µg) 1 hour before the subcutaneous inoculation of A20 tumor cells. Tumor growth curves of five mice per group are shown. WT (FIG. 8B) and γ^{-/-} (FIG. 8D) mice were inoculated with B6BL-CD40 tumor cells and treated with the indicated CD40 antibodies or control IgG (d3: 200 µg; d6: 200 µg) with or without αCD8-depleting antibodies (clone 2.43). Survival curves of four to six mice per group are shown in FIG. 8B and D. As shown in FIG. 8C, WT mice (three per group) were treated with 200 µg of the indicated CD40 antibodies or control IgG. Six days later, the percentage of CD40⁺ cells and the ratio of CD8⁺ to CD4⁺ T cells were analyzed in peripheral blood and presented in the bar graph. Error bars are SD. **P < 0.01, ***P < 0.001. Aone-way ANOVA with a Dunnett post hoc test [(A) and (C)] or log-rank test [(B) and (D)] was used to compare as follows: all groups to the mIgG control groups, and αCD40:mIgG1 to αCD40:mIgG2a in (C).

### Example 7 - Anti-tumor effect of αDR5

TNFR family members can be classed into two major groups based upon the signaling properties of their cytoplasmic domains: members containing death domains (death receptors) and members containing TNF receptor-associated factors (TRAF) interacting motifs (Aggarwal, Nat Rev Immunol. 2003 Sep;3(9):745-56). In this example, assays were carried out to confirm that FcγRIIb supports members of both groups. DR5 and CD40 belong to the aforementioned two major groups, respectively. As FcγRIIb requirement for agonistic CD40 antibody activities has been shown above, agonistic DR5 antibodies were studied to test whether the FcγRIIb requirement for agonistic CD40 antibody activities applies to the other major group and therefore the TNFR family in general.

DR5 ligands, the TNF-related apoptosis inducing ligands (TRAIL), and their apoptosis inducing receptors (TRAIL-R1/DR4 and TRAIL-R2/DR5 in human, TRAIL-R/DR5 in mice) are implicated in anti-tumor response. Both TRAIL and agonistic DR5 mAbs have being actively tested in animal models and clinical trials. An agonistic hamster anti-mouse DR5 antibody, MD5-1, has been shown to induce cholestatic liver damage and mediate anti-tumor response, by triggering apoptotic pathways in cholangiocytes and tumor cells, respectively (Takeda et al., Proc Natl Acad Sci USA. 2008 Aug 5;105(31):10895-900; Haynes et al., J Immunol. 2010 Jul 1;185(1):532-41).

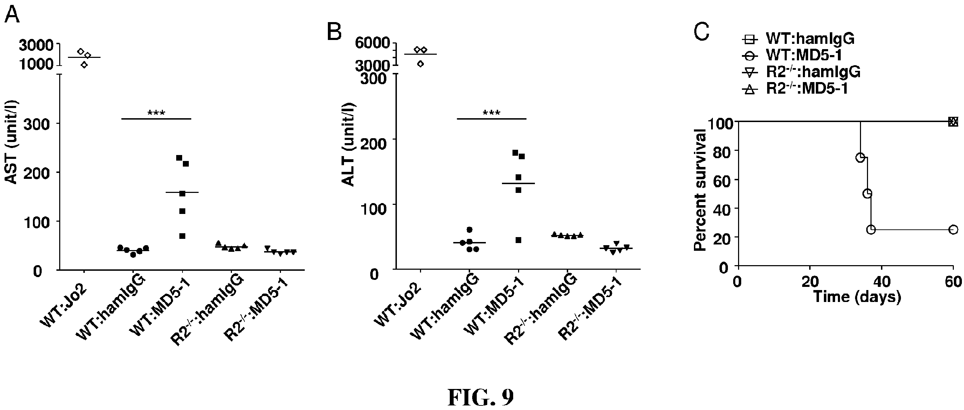

Briefly, the involvement of FcγRIIb in MD5-1 induced liver damage was first tested. Wild-type C57BL/6 and FcγRIIb-deficient (R2^{-/-}) mice were treated hamster IgG (hamIgG) or MD5-1 through intravenous injection on day 0, 3, 6, or 9. Two biomarkers, serum aspartate transaminase (AST) and alanine transaminase (ALT) were measured to assess liver damages on day 14. The results were shown in FIGs. 9A and B, respectively. Survival was also monitored for two month and the results were shown in FIG. C. Mice treated with Jo2 (anti-Fas) for 2 hours were included in the AST and ALT tests as positive controls. In FIGs. A and B, short horizontal lines are mean values; *** *p* < 0.001, one-way analysis of variance (ANOVA) with Tukey post-hoc test.

As shown in FIG. 9, in wild-type C57BL/6 mice, MD5-1 treatment resulted in elevated AST and ALT levels (FIGs. 9A and B), and mortalities (FIG. 9C). In contrast, MD5-1 treated FcγRIIb-deficient mice (R2^{-/-}) were protected, demonstrating that agonistic DR5 antibodies require FcγRIIb to induce liver toxicity.

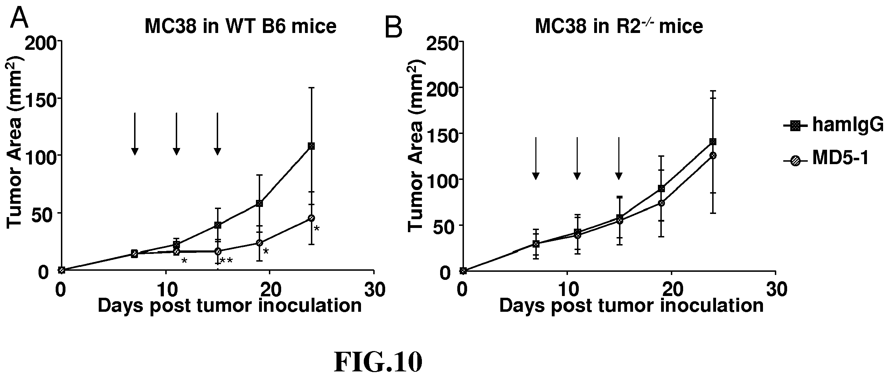

FcγRIIb-requirement was also tested for the anti-tumor activities of MD5-1 using a DR5-sensitive syngeneic tumor model, MC38. More specifically, wild-type C57BL/6 and FcγRIIb-deficient (R2^{-/-}) were inoculated with 10⁶ MC38 cells subcutaneously, and treated with hamster IgG (hamIgG) or MD5-1 antibodies through intravenous injection, on days 7, 11, and 15. Growth curves were obtained and presented in FIG. 10, where arrows represent treatments; * *p* < 0.05, ** *p* < 0.01, two-tailed *t* test; Error bars are S.D.

As shown in FIG. 10A, MD5-1 significantly inhibited MC38 growth in wild-type C57BL/6 mice. In contrast, the anti-tumor activities of MD5-1 were abolished in FcγRIIb-deficient mice (FIG. 10B). Taken together, these data demonstrated that FcγRIIb is required for the activities of agonistic DR5 antibodies.

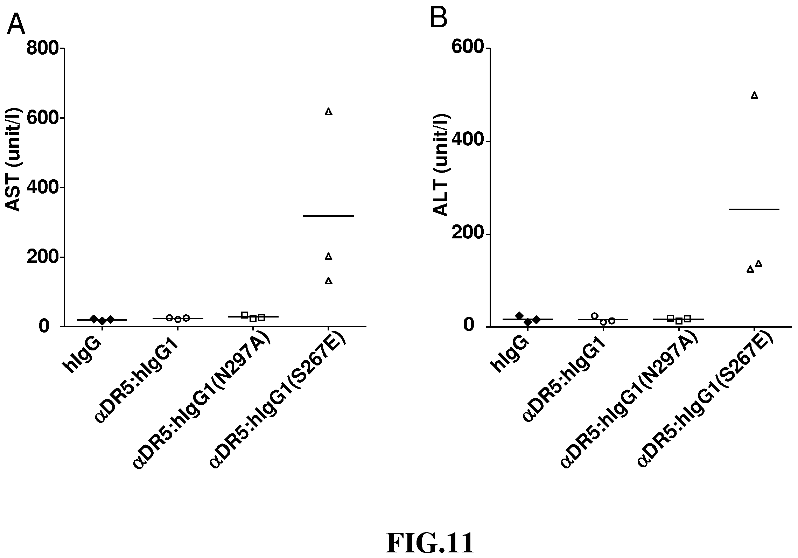

In order to explore whether activities of agonistic DR5 antibody can be manipulated through FcγRIIb-targeted Fc-engineering, the hamster antibody heavy and light variable region genes (V_{H} and V_{L}, respectively) were cloned from MD5-1 hybridoma. Chimeric DR5 antibodies with unmutated human IgG1 Fc (hamster V_{H} and human Cγ1 for the heavy chain, and hamster V_{L} and human C_{κ} for the light chain), its FcγR-null variant (N297A), and a human FcγRIIb-enhanced variant (S267E) were produced. These DR5 antibodies were tested to induce liver toxicity in human FcγRIIb-transgenic mice ((γR2)^{-/-}hRIIb⁺). Briefly, human FcyRIIb-transgenic mice ((γR2)^{-/-}hrIIb⁺) were treated human IgG, MD5-1-derived antibodies with human IgG1 Fc, its N297A, or S267E variants through intravenous injection. Serum AST and ALT levels were analyzed on day 7, and the results were presented in FIGs. 11A and B, respectively, where short horizontal lines are mean values.

As shown in FIG. 11, while both the unmutated and N297A versions of chimeric human IgG1 DR5 antibodies failed to induce detectable liver toxicity, the S267E variant with enhanced human FcγRIIb binding was potent in inducing liver damage. These data demonstrated that the activities of DR5 antibodies can be enhanced by increasing FcyRIIb-binding.

In summary, studies of agonistic antibodies of two TNFR members with distinct signaling pathways, CD40 and DR5, have demonstrated that FcγRIIb is a common requirement for the activities of agonistic TNFR antibodies. The adjuvant and anti-tumor activities of agonistic CD40 antibodies, the liver toxicity effects and anti-tumor activities of agonistic DR5 antibodies, all require FcγRIIb. These activities can be enhanced or attenuated by increasing or decreasing the FcγRIIb-binding affinities. FcγRIIb-targeted Fc-engineering is, therefore, a novel strategy to optimize therapeutic effects of agonistic antibodies targeting TNFR family members.

All publications cited in the specification, both patent publications and non-patent publications, are indicative of the level of skill of those reasonably skilled in the art to which this invention pertains. All these publications are herein fully incorporated by reference to the same extent as if each individual publication were specifically and individually indicated as being incorporated by reference.

Although the invention herein has been described with reference to particular embodiments, it is to be understood that these embodiments are merely illustrative of the principles and applications of the present invention. It is therefore to be understood that numerous modifications may be made to the illustrative embodiments and that other arrangements may be devised without departing from the spirit and scope of the present invention as defined by the following claims.

## Claims

1. A method for obtaining an antibody variant having enhanced agonistic activities and enhanced T cell stimulation and activation activity as compared to a starting antibody, said starting antibody being an agonistic antibody against a TNFR superfamily receptor, the method comprising:
providing a first nucleic acid sequence encoding a Fc region of the starting antibody;
modifying the first nucleic acid sequence to obtain a second nucleic acid encoding a mutant Fc region having one or more mutations, said one or more mutations causing a higher binding affinity to an inhibitory Fcγ receptor, as compared to the starting antibody, and
generating a modified antibody that is identical to the starting antibody except that the modified antibody has the mutant Fc region, thereby obtaining the antibody variant,
wherein (i) the mutant Fc region is a human IgG1 Fc region and (ii) the one or more mutations include one or more selected from the group consisting of S267E, G236D, S239D, L328F, and I332E.

2. The method of claim 1, wherein the inhibitory Fcγ receptor is human FcγRIIb.

3. The method of claims 1 or 2, wherein the TNFR superfamily receptor is CD40 or DR5.

4. The method of any one of claims 1-3, wherein the modified antibody exhibits a reduced antibody-dependent cell-mediated cytotoxicity (ADCC) as compared to the starting antibody.
